Europäisches Patentamt

European Patent Office

Office européen des brevets

(11) Publication number: **0 205 506 B1**

## EUROPEAN PATENT SPECIFICATION

(45) Date of publication of patent specification: **11.03.92**

(51) Int. Cl.5: **C12N 15/80**, C12P 1/02, //C12R1:785

(21) Application number: **86900082.8**

(22) Date of filing: **13.12.85**

(86) International application number: **PCT/DK85/00120**

(87) International publication number: **WO 86/03774 (03.07.86 86/14)**

(54) **A METHOD OF TRANSFORMING FUNGI WITH A VECTOR.**

(30) Priority: **14.12.84 DK 6019/84**

(43) Date of publication of application: **30.12.86 Bulletin 86/52**

(45) Publication of the grant of the patent: **11.03.92 Bulletin 92/11**

(84) Designated Contracting States: **AT BE CH DE FR GB IT LI LU NL SE**

(56) References cited:
EP-A-10 099 226
US-A- 4 082 613

Journal of Bacteriology, Vol 159, Sept 1984, Linz J. et al., "Differential gene expression during aerobic germination of Mucor racemosus sporangiospores", p 965-972

Proc. Natl. Acad. Sci, USA, Vol 80, Feb 1983, Stohland L. et ., "Construktion of a shuttle vector for the filamentous fungus Neurospora crassa". p 1058-1062

(73) Proprietor: **CHR. HANSEN'S LABORATORIUM A/S**
**Boge Allé 10-12**
**DK-2970 Horsholm(DK)**

(72) Inventor: **VON WETTSTEIN, Diter**
**Aasevej 13**
**DK-3500 Vaerlose(DK)**
Inventor: **VAN HEESWIJCK, Robyn**
**Grabynkevej 13**
**DK-2700 Bronshoj(DK)**
Inventor: **RONCERO, Maria, Isabel, Gonzalez**
**Cl-Enmedio 11-1'-1'**
**14004 Cordoba(ES)**

(74) Representative: **Patentanwälte Grünecker, Kinkeldey, Stockmair & Partner**
**Maximilianstrasse 58**
**W-8000 München 22(DE)**

Proc. Natl. Acad. Sci, USA, Vol 79, June 1982, Stahl U et al., "Replication and expression of a bacterial-mitochondrial hybird plasmid in the fungus Podospora anserina p 3641-3645

Chemical abstracts, vol. 102 (1985), abstract n 161 420b, Carlsberg Res Commun., 1984, 49(7), 691-702 (Eng)

## Description

The present invention relates to a method of transforming a fungus belonging to the class Zygomycetes with a recombinant expression vector by means of recombinant DNA technology.

Most recombinant DNA work has been performed in bacteria, such as *Eschericia coli*, and therefore most efforts to transfer the knowledge developed by this basic research to areas of industrial utility have been centered around attempts to achieve industrial production of gene products expressed in *E. coli* or other bacteria such as *Bacillus subtilis* and, in some cases, in yeasts, principally *Saccharomyces cerevisiae*. However, the use of these organisms has certain drawbacks, principally because of difficulties in achieving expression of eucaryotic genes in these organisms, of maintaining the vector plasmids in the cells, and/or because of the problem of recovering the expressed gene product from the culture.

It has now been found that certain fungi, more specifically filamentous fungi belonging to the class Zygomycetes, possess certain valuable properties which may contribute to solving one or more of the problems outlined above.

A. Several species belonging to the class Zygomycetes, in particular those belonging to the genus *Mucor*, have been utilized industrially for a number of years, principally to produce proteolytic enzymes used as milk coagulants in the production of a variety of cheeses. Large-scale production of these organisms which are grown in submerged culture or on a semisolid medium, is therefore well known with respect to optimal growth conditions such as the composition of the nutrient medium in which the culture is grown.

B. Fungi of the class Zygomycetes used in industrial production are non-pathogenic and therefore do not constitute an environmental hazard.

C. It is well known that at least certain products such as the proteolytic enzymes mentioned above, are excreted by the organisms into the culture medium. It is contemplated that the mechanism by which the fungi excrete these proteins may be utilized in various ways so as to obtain extracellular production of the gene products of the inserted genes which would greatly facilitate recovery procedures and therefore reduce the cost of the end product.

D. It is known that certain genes (from these or other fungi) are not expressed in bacteria such as *E. coli* or yeasts such as *Saccharomyces cerevisiae*, possibly due to existence of introns in the DNA sequence or the presence of alternative transcription, messenger RNA processing (e.g. exon splicing) or translation signals. It is contemplated that such genes might be more easily expressed when inserted in fungi of the class Zygomycetes, such as *Mucor* species. It is also anticipated that certain eucaryotic genes which are difficult or impossible to express in bacteria or yeasts for these or other reasons may more easily be expressed in zygomycetous fungi.

It has hitherto not been possible to achieve transformation of Zygomycetes species, i.e. the direct incorporation of selected genes into the host organism by means of a plasmid vector. It has even been difficult to obtain an adequate genetic analysis of Zygomycetes species by recombination studies due to the difficulties in utilizing their sexual cycle. Mating of (+) and (-) strains does not always result in the formation of zygospores, and in some species the numbers produced are low, while in others their development remains incomplete (cf. M.A.A .Schipper, *Studies in Mycology 17*, 1978, 1-52). Moreover, the successful germination of zygospores under laboratory conditions is rare and is preceded by a considerable period of dormancy (cf. W. Gauger, *Mycologia 57*, 1965, pp. 634-641).

The present invention has overcome these difficulties by providing a method by which genetic material may be introduced into a host organism belonging to the class Zygomycetes.

Accordingly, the present invention relates to a method of transforming a fungus of the class Zygomycetes, in which sporangiospores or germlings of a fungus of the class Zygomycetes are transformed with a recombinant expression vector which is replicated in the fungal species, the transformation being effected by treating germinating sporangiospores with one or more suitable lytic enzymes, transforming the resulting protoplasts with an expression vector, and regenerating the cell wall.

In the present context, the term "sporangiospores" denotes vegetative, asexual spores of Zygomycetes species, end the term "germlings" denotes germinating spores.

It has surprisingly been found that, by suitable treatment, as described in further detail below, sporangiospores end germlings may be brought to a condition in which they are capable of receiving external genetic material. This means, that by the method of the invention, it has become possible to obtain direct cloning of specific genes in fungi of the class Zygomycetes by the transformation methods usually employed in recombinant DNA technology, including the use of a recombinant expression vector.

The fungus employed for transformation purposes is preferably a fungus of the genus *Mucor* as these fungi have been used extensively for industrial purposes and, therefore, the conditions under which they should be cultivated in large-scale production are well known. The *Mucor* strain selected for transformation may be a mesophilic *Mucor* species such as *M. circinelloides*, *M. racemosus* or *M. rouxii*, or a thermophilic *Mucor* species such as *M. miehei* or *M. pusillus*.

One of the principal difficulties in obtaining transformation of fungi of the formation of protoplasts requires the removal of the cell wall by digestion with one or more lytic enzymes. It has for instance been found that the vegetative spores (sporangiospores) of *Mucor* may be a less suitable source of protoplasts due to the extreme resistance of the cell wall to lytic digestion. The high content of melanin (10% by weight of dry matter) is implicated in this resistance as it is a known inhibitor of cell wall lytic enzymes (A.T. Bull, *Arch. Biochem. Biophys. 137*, 1970, pp. 345-356). In accordance with the present invention, however, a method has been developed whereby the transformation is effected by treating germinating sporangiospores at a unique developmental stage with one or more suitable lytic enzymes, transforming the resulting protoplasts with the appropriate vector, and regenerating the cell wall.

The treatment of the germinating sporangiospores according to this method has become possible because of the finding that, during germination, a vegetative wall is formed *de novo* underneath the sporangiospore wall, later emerging to become the germ tube wall. The lack of continuity between spore and vegetative walls is correlated with marked differences in their composition, including the absence of melanin in the latter. Instead, chitin and chitosan have become the main components; in one *Mucor* species, *M. rouxii*, for example, they constitute 9.4% and 32.7% of the hyphal cell wall, respectively. It has further been found that to degrade this cell wall, lytic enzymes such as chitosanase may be employed. Chitosanases are not yet commercially available but are produced as extracellular enzymes by a variety of microorganisms. The culture fluids from some of these microorganisms, or the chitosanases purified therefrom, can digest the hyphal cell walls of for instance *Mucor* and enable the formation of protoplasts from *Mucor* and the related genus *Phycomyces*. Chitinase which is commercially available may also be employed, but not as the sole lytic enzyme as it is unable or only partially able to hydrolyze chitosan.

One culture fluid which has been found useful for the present invention is the culture fluid, optionally in concentrated form, of *Streptomyces* sp. No. 6, also termed "streptozyme" which contains chitosanase in unpurified form and possibly also other lytic enzymes. It is also possible to add an enzyme which in itself has little or no lytic effect, but which increases the effect of chitosanase. In experiments it has been found that when certain commercially available polysaccharases are employed together with chitosanase or the unpurified streptozyme, this results in increased yields of protoplasts, even when used in combination with a low concentration of the lytic enzyme proper, although none of these commercially available enzymes in themselves have any visible lytic effect.

It has been found that an increase in the concentration of the lytic enzyme employed to digest the cell walls, increases the rate of protoplast formation and the final yield of protoplasts obtained. When comparing different batches of, e.g., streptozyme, their efficacy in producing protoplasts in *Mucor* species is directly proportional to the total chitosanase activity present. Thus, 0.5 mg/ml of a batch with a specific chitosanase activity of 2.3 produces equivalent results to 1.0 mg/ml of a batch with a specific chitosanase activity of 1.2.

It has also been found by experimentation that the formation of protoplasts from germinating sporangiospores is to some extent dependent on the stage of germination as the resistance to lytic enzyme seems to be reacquired, though to a lesser extent, as the hyphae age and grow in length. In fact, the yields of protoplasts obtained tend to be highest when the germlings had a germ tube length of 35-50 $\mu$m. Below this size, digestion of the cell wall at the growing hyphal tip still occurs, but the amount of material extruded decreases, with a consequent reduction in the number of protoplasts formed. When the germ tube length exceeds approximately 60 $\mu$m, the amount of hyphal structure visibly resistant to lytic enzyme action increases, the protoplast yield decreases, and the homogeneity of the protoplasts obtained also decreases (as evidenced by a greater variation in size, refractility and apparent membrane integrity). Therefore, it is preferred that, in order to obtain a high yield of morphologically homogeneous protoplasts, the germlings to be treated with the lytic enzyme be relatively homogeneous with respect to extent of germination (i.e. germ tube length). Synchronous germination can be induced in the genus *Phycomyces* by various treatments such as heat shock; in another genus, *Rhizopus*, synchronous germination may be induced by means of a phosphate buffer, pH 6.5, containing proline. However, although a specific induction method for *Mucor* has yet to be developed, there are certain indications that a more synchronous germination may also be obtained in *Mucor*, for instance by germinating freshly harvested sporangiospores in complete media instead of using frozen spores or minimal media, and by incubating the sporangiospores at a temperature which approaches the optimal growth temperature of the species in question, this latter effect having been observed for thermophilic species in particular.

In some cases, it is necessary to employ osmotic stabilizers during protoplast formation. It is important that the osmotic stabilizer employed does not inhibit the activity of the lytic enzyme preparation to the effect that no cell wall degradation takes place. Solutions of sugar alcohols such as sorbitol and mannitol tend to be the most effective osmotic stabilizers, while the use of salt solutions, e.g. KCl, MgCl$_2$ (NH$_4$)$_2$SO$_4$ or MgSO$_4$ seems to result in no protoplast formation at all. When employing sorbitol as the osmotic stabilizer when forming protopasts from *Mucor* germlings, the optimal concentration was found to be 0.35-0,5 M. At concentrations of 0.65 M or higher, no cell wall degradation is observed, and at concentrations of 0.3 M or lower, the action of the lytic enzyme tends to be accompanied by extensive cell lysis rather than protoplast formation.

The pH of the medium used during incubation should preferably be in the range of 4.5-7.5, so as not to interfere with the activity of the lytic enzyme employed.

Regeneration of the cell wall after transformation may be effected by washing the protoplasts twice in a suitable buffer or growth medium containing a suitable osmotic stabilizer such as sorbitol, and then plating the protoplasts on a solid agar medium also containing the osmotic stabilizer. The plating of the protoplast suspension may be performed by spreading the protoplasts directly on the surface of the solid agar medium, or preferably by mixing them into an agar overlay containing the osmotic stabilizer which is held in a molten condition and then poured onto the surface of the solid agar medium. Embedding the protoplasts in agar permits cell wall regeneration to occur reproducibly and at high frequencies.

In accordance with the invention, the transformation procedure should preferably include a method of selecting the transformed cells. Accordingly, the fungal strain of the class Zygomycetes may conveniently be a suitable auxotrophic mutant strain (i.e. a strain which requires one or more growth factors not needed by the wild type strain) or a mutant strain which is sensitive to a certain antibiotic which strain, when transformed, is complemented by an expression vector carrying the counter-selectible prototrophic or antibiotic resistance gene.

Auxotrophic mutants have been found to be particularly useful in the method of the present invention. Such mutant strains may be produced by conventional mutagenization methods, including ultraviolet radiation, ionizing radiation or treatment with a chemical mutagen. The desired auxotrophic mutants are then isolated by selective elimination of the prototrophic survivors of the mutagenic treatment. Most such isolation methods are based on the fact that metabolically active cells are more sensitive to antibiotics and physical agents than inactive cells, such as ungerminated spores. Therefore, a preferential killing of prototrophs is the result when mutagenized cultures grown in minimal medium, i.e. a medium which does not contain the growth factors needed for the growth of auxotrophs, are exposed to a counter-selective agent. Other methods include differential freeze-killing of germinating versus non-germinating spores, or differential heat sensitivity of germinated prototrophic spores as opposed to ungerminated auxotrophic spores. The most advantageous method in the present context has been found to be an approach based on the use of an antibiotic which induces considerable changes in permeability by interaction with the sterols in the cell walls of metabolically active cells. A particularly advantageous antibiotic is a polyene antibiotic such as N-glycosyl-polifungin (NGP) which has the added advantage of being water-soluble unlike most other polyene antibiotics such as nystatin.

Utilizing the enrichment method described above, it is contemplated that successive treatments with antibiotic may lead to a further increase in the frequency of mutants obtained. Such additional cycles of counter-selection may be advisable when specific and rare phenotypes of mutants are needed. A further selection of auxotrophic mutants may be obtained by growing colonies which have survived the antibiotic treatment and selecting those colonies which fail to grow on a medium which does not contain specific growth factors, but which grow on medium containing these growth factors.

Although the use of auxotrophic mutants is preferred, in particular where *Mucor* species are concerned, as *Mucor* shows a low intrinsic sensitivity to most of the antibiotics for which genes conferring resistance from other organisms are available, it is, however, also possible to employ an antibiotic sensitive mutant strain. In both cases, the expression vector used to transform the fungal cells should carry a gene which complements this property of the host organism, i.e. a gene which either mediates resistance to the antibiotic to which the strain is sensitive or which mediates prototrophy to the auxotrophic mutant so that it reverts to the wild type nutrient requirement.

In accordance with the invention, the expression vector used to transform the host cells may either be self-replicating, i.e. it may exist as an extrachromosomal entity the replication of which is independent of chromosome replication (i.e. a plasmid), or the vector may be integrated stably into one or more of the host chromosomes by insertion, for instance by homologous recombination (the vector is so constructed that at least a portion of it corresponds to - is homologous to - a portion of the chromosome; this results in a "joining" of the vector portion to the chromosome) and replicated concurrently with these. When the vector is an autonomously replicating plasmid, it carries an origin of replication which is capable of functioning in the host cell so that the plasmid is replicated in such a way that it is maintained in the cell.

In some cases, it is preferred that the vector is a shuttle vector which can be replicated in a fungal species of the class Zygomycetes, in *E. coli* and/or in a yeast such as *Saccharomyces cerevisiae*. A shuttle vector may be advantageous as it facilitates the tranfer of genes already cloned in *E. coli* or yeast vectors and the construction, multiplication and analysis of vectors containing new or additional genes. Specific examples of such vectors are pMCL 1302 which is prepared according to the invention and, in Example 8 below, has been introduced in *Mucor circinelloides*, leucine auxotroph R7B. The strain carrying pMCL 1302 is deposited in the Centraal Bureau voor Schimmelkulturen under the Accession No. CBS 754.84. Another vector, produced in similar manner, is pMCL 1647 which has been introduced in the same *Mucor* strain. The strain of *Mucor circinelloides* R7B carrying pMCL 1647 is also deposited in the Centraal Bureau voor Schimmelkulturen under the Accession No. CBS 755.84.

In another aspect, the present invention relates to an expression vector for transformation of fungi of the class Zygomycetes, which carries a DNA sequence of zygomycetous DNA, a DNA sequence coding for a desired gene product, and a marker for the identification and/or selection of cells transformed with the vector, and which is replicated in fungi of the class Zygomycetes. In the present context, the term "DNA sequence" denotes a sequence of basepaired nucleic acids which include one or more structural genes as well as the appropriate transcriptional and translational start and stop signals, promoters, convenient restriction sites, etc. As described above, the selective marker may be a gene mediating antibiotic resistance to an antibiotic sensitive fungal strain or a gene mediating prototrophy to an auxotrophic fungal strain. In the latter case, the gene may be one which, in its absence or in modified form, causes the organism to require the presence of a specific amino acid or several amino acids, one or more nucleotides or vitamins in the growth medium, and when this gene is complemented by the introduction of the vector into the host organism, the compound or compounds in question are no longer required for growth.

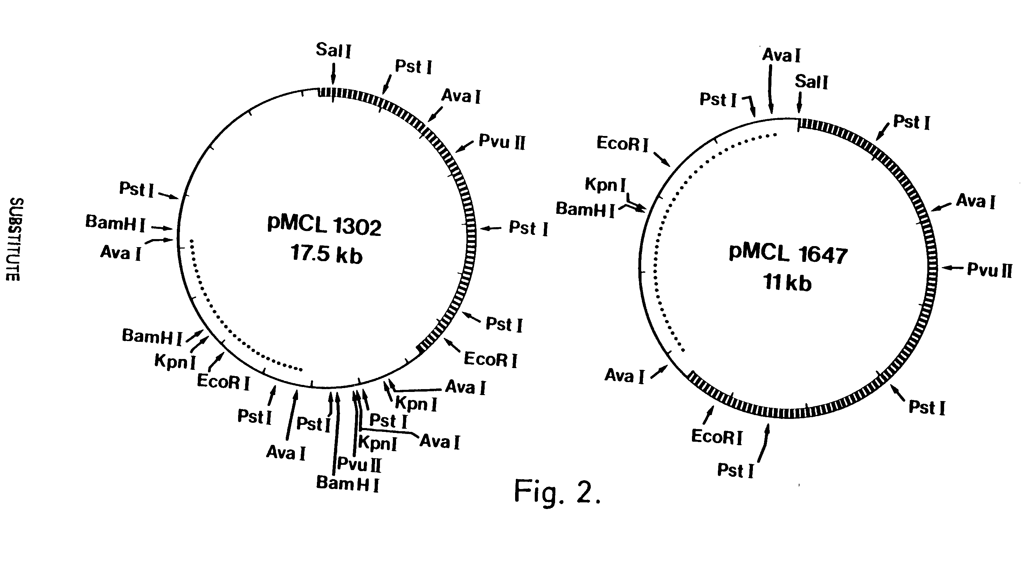

The invention further relates to an expression vector for transformation of a fungus of the class Zygomycetes. The vector comprises a sequence of zygemycetous DNA, a marker for the identification and/or selection of cells transformed with the vector, and at least one restriction site for the insertion of a DNA sequence coding for a desired gene product. The vector is replicated in fungi of the class Zygomycetes. The restriction site should preferably be located on the vector at a site where the insertion of the DNA sequence coding for the desired gene product will not interfere with the replication of the vector and/or selection of the host organism to which the vector is to be transferred, that is, the restriction site should be located outside the origin of replication and the DNA sequence coding for the selective marker. Examples of vectors which satisfy these requirements are pMCL 1302 and pMCL 1647 which are further described and characterized in the Examples and which are shown in Fig. 2.

The present invention also relates to a method of preparing an expression vector as described above, which comprises inserting, in a plasmid which is replicated in a fungus of the class Zygomycetes, a DNA sequence for a marker for the identification and/or selection of cells transformed with the vector, providing at least one restriction site for the insertion of the DNA sequence coding for a desired gene product at a location where the DNA sequence does not interfere with replication and/or selection, and inserting the DNA sequence for the desired gene product at this site. If a convenient restriction site is not available, it may be possible to provide it in the form of a linker inserted at a suitable site. The procedures employed to produce the expression vector of the invention may be those conventionally employed for this purpose in the field of recombinant DNA technology.

The present invention additionally relates to a fungus of the class Zygomycetes which carries a recombinant expression vector which is replicated in the organism. Genetic transformation, i.e. the incorporation of isolated genes in the form of isolated DNA, has previously only been achieved in filamentous fungi belonging to the class Ascomycetes, namely *Neurospora crassa* (Case et a., *Proc. Natl. Acad. Sci. USA 76*, 1979, p. 5259), *Podosporo anserina* (Stahl et al., *Proc. Natl. Acad. Sci. 70*, 1982, p. 3641) and *Aspergillus nidulans* (Balance et al., *Biochem. Biophys. Res. Commun. 112*, 1983, p. 284).

Finally, the present invention relates to a method of producing a gene product from a fungus of the class Zygomycetes, in which sporangiospores or germlings of a fungus of the class Zygomycetes are transformed with a recombinant expression vector which is replicated in the fungal species and which carries a DNA sequence coding for a desired gene product, the transformed sporangiospores or germlings are grown in a suitable culture medium to express said DNA sequence, and the resulting product is harvested.

The transformed sporangiospores or germlings are grown in a suitable culture medium to express the DNA sequence. The cultivation is suitably performed using conventional techniques well known for largescale production of Zygomycetes species, in particular *Mucor* species, such as the techniques described by, e.g., K. Aunstrup, "Proteinases", in A.H. Rose, ed., *Microbial Enzymes and Bioconversions*; *Economic Microbiology*, Vol. 5, Academic Press, New York, 1980, pp. 50-114, including the use of conventional nutrient media which are known to be suitable or optimal for the fungal species in question. For instance, *Mucor* species may be grown in submerged culture or on a semisolid medium. For submerged cultivation, it is preferred that the nutrient medium is relatively concentrated (with a dry matter content of up to about 10-15%) and that it has a high content of protein. The medium may be supplied with carbohydrates such as starch, starch derivatives or sugars, preferably in such a way that the carbohydrate concentration is kept low at all times (a so-called fed-batch process). For semisolid fermentation, a medium such as bran (for instance wheat bran) may be moistened to a dry matter content of about 50% inoculated with spores of the organism producing the desired product and left under aeration. The growth conditions may further include an adaptation to the optimal growth temperature of the fungus as well as certain specific nutrients such as mineral salts or trace minerals.

The harvesting of the resulting gene product may be performed by conventional methods in the manner most convenient to the production process employed, dependent, *inter alia*, on the type of product being produced, the end use of this product and, most importantly, whether the product is excreted into the medium or not. Most usually, when dealing with excreted products which are found in soluble form in the medium, the solids (including residues of the medium components and host organisms) are removed by filtration and centrifugation (cf. for instance K. Aunstrup, "Production, Isolation and Economics of Extracellular Enzymes", in L.B. Wingard et al. (eds.), *Applied Biochemistry and Bioengineering*, Vol. 2, Academic Press, New York, 1979, pp. 28-69.

When the fungus employed for the production of the gene product is a fungus of the genus *Mucor*, the growth conditions optimal for the cultivation of *Mucor* species include the presence of proteinaceous nutrients such as soy protein, casein, whey powder, brewer's yeast or yeast extract, carbohydrate-containing nutrients such as ground grain, e.g. corn or barley, starch, e.g. corn or potato starch, or starch hydrolysates, or sugars such as glucose, lactose or sucrose, and salts such as phosphates, magnesium salts, carbonates, sulphates, ammonium salts or nitrates, and trace minerals such as Zn, Fe or Cu. The specific composition of the medium depends, to some extent, on the product to be produced. Specific media suitable for the production of, e.g. enzymes, are described in, e.g., US Patent No. 3,988,207. Other factors influencing the growth of the organisms include the growth temperature.

As mentioned above, one of the valuable properties of fungi of the class Zygomycetes, especially of the genus *Mucor*, is their ability to excrete certain of their products, such as certain metabolites and enzymes, into the culture medium which, for instance, is the case with certain enzymes such as acid proteases and other enzymes. In a preferred embodiment of the present invention, this process is utilized so that the product of the DNA sequence inserted in the fungal cells is excreted into the medium by the natural route of these organisms and recovered therefrom by established industrial procedures.

Thus, in order to obtain excretion of the product, it may be contemplated to integrate the expression vector into the host chromosome at the site of a gene the product of which is known to be exported by the organism into the culture medium. The integration may be directed, for instance by homologous recombination, to occur in such a manner that the expression of the introduced gene utilizes the signals of the endogenous gene which it has replaced, including those responsible for the eventual export of the gene product into the culture medium.

One of the advantageous features of the method of the present invention resides in the fact that products exported by the host cells into the medium may be recovered therefrom by simply filtering or centrifugating off the host organisms together with residues of the medium components, without further purification being necessary. This, of course, greatly reduces the cost of the product in question.

For some special applications requiring a high degree of purity, however, the product may be subjected to various purification procedures according to need, for instance further filtration, precipitation in a manner known *per se* (usually with water-soluble organic solvents such as acetone or certain alcohols, or with inorganic salts such as ammonium sulphate or sodium sulphate). If a further purification is needed, the partially purified product may be redissolved and subjected to various known affinity chromatographic methods such as gel filtration or ion exchange chromatography. The purification process may be accelerated by employing high performance liquid chromatography. The mode and degree of purification depends on the product produced; thus, if the product is one which is to be used industrially, it may not be necessary to perform further purification than the basic recovery procedure by filtering off the host organisms.

The gene product to be produced by the method of the invention from the Zygomycetes species may be any product conveniently produced by recombinant DNA technology. Thus, the product may be a polypeptide protein or fragment thereof, an enzyme or a non-proteinaceous product of reactions of enzymes with a compound in the culture medium, or a low molecular weight product such as hormones or nucleic acids. Products of particular interest are products which are either known or expected to be expressed in or exported by other organisms such as bacteria or yeasts with great difficulty, if at all. Such products may comprise products from other fungal genera, the industrial fermentation of which is less well developed than that of fungi of the class Zygomycetes, in particular *Mucor* species, or genes of higher eucaryotes. Interesting gene products are enzymes, including lipase, amyloglucosidase, $\alpha$-amylase, $\beta$-galactosidase, cellulase or proteases such as chymosin. Some of these gene products may be produced by certain Zygomycetes species (e.g. *Mucor* species), but may be produced in insufficient amounts, or may have undesirable properties such as excessive thermostability or thermolability, thus requiring a modification procedure to be industrially useful; it may also be desirable to confer a different specificity to the gene products by genetic alteration. The DNA sequence for the modified gene product may be inserted into a vector and introduced and expressed in a host organism, for instance in such a way that it replaces the inherent DNA sequence for the natural product, in accordance with the method of the invention.

It should be noted that the gene encoding chymosin (calf rennet) which is extensively used as a milk coagulating enzyme in the production of cheese, has been introduced and expressed inside cells of *Escherichia coli* (cf. Nishimori et al., *Gene 29*, 1984, p. 41; Emtage et al., *Proc. Natl. Acad. Sci. USA 80*, 1983, pp. 3671-3675) and inside cells of the yeast *Saccharomyces cerevisiae* (Mellor et al., *Gene 24*, 1983, p. 1; Goff et al., *Gene 27*, 1984, p. 35). Even when expression is obtained in these organisms, it is therefore necessary to disrupt the cells in order to obtain the product after which the cell debris must be removed. Such a purification procedure is not necessary when introducing the same gene into species of the class Zygomycetes in such a way that the gene product is excreted, as outlined above.

The method of the present invention of producing a gene product from a fungus of the class Zygomycetes by transforming zygomycetous sporangiospores or germlings with an expression vector of the invention, is distinguished from the known genetic transformation methods used in connection with filamentous fungi in the following way:

It allows direct cloning of genes by complementation of suitable mutations in the recipient strain with plasmid DNA containing inserts from a genomic library of the wild-type strain. The plasmid may carry other genes to be expressed in the recipient strain. This is opposed to the known methods employed with filamentous fungi for the cloning of specific genes which use indirect means such as screening genomic libraries for the complementation of mutants in *E. coli* or *S. cerevisiae*. This is not applicable to Zygomycetes species as zygomycetous genes cannot be functionally expressed in these two hosts generally employed for recombinant DNA work. The *in vitro* techniques of complementary DNA synthesis from messenger RNA and hybridization to synthetic oligonucleotide probes employed with the ascomycetous fungi *Aspergillus nidulans and Neurospora crassa* and which might also be applicable to zygomycetous fungi are more cumbersome than the method of the present invention. The present invention is in fact believed to constitute the first instance of genetic transformation of fungi of the class Zygomycetes. The expression vector incorporated in the fungus may be one of those described above, and the fungus is preferably a fungus of the genus *Mucor*, such as an auxotrophic mutant strain as described above.

The insertion of a DNA sequence coding for a desired gene product into the expression vector may be carried out in the following way:

The expression vector is digested with one or more restriction enzymes so that the circular molecule is opened at one position. The DNA sequence to be inserted is also digested with one or more restriction enzymes to produce ends which may be ligated to the ends of the expression vector, using the enzyme $T_4$-DNA ligase, either directly (in the case of complementary "cohesive" ends) or after the creation of "blunt" ends by treatment with DNA Polymerase 1 (Klenow fragment) or S1 nuclease. The site of insertion of the DNA sequence coding for the desired gene product is so selected that it follows a promoter sequence known to function in *Mucor*. An example of the latter is the promoter sequence responsible for the expression of the Leu$^{+}$ gene of *Mucor circinelloides* which is known to be found in the 2.1 kb *Ava*I-*Kpn*I fragment of the *Mucor* DNA insert of plasmid pMCL 1302.

The invention is further described with reference to the drawings in which

Fig. 1 shows a restriction map of the parent plasmid YRp17 used for constructing expression vectors of the present invention. In the Fig. 1, the dotted areas denote DNA from *E. coli*, and the filled-in areas denote DNA from *S. cerevisiae*. The arrows denote the direction of transcription.

Fig. 2 shows restriction maps of two expression vectors according to the invention, pMCL 1302 and pMCL 1647 (not drawn to the same scale). The thick lines denote YRp17 DNA (hatched areas denote DNA from *E. coli* and the filled-in areas denote DNA from *S. cerevisiae*), and the thin lines denote the *Mucor* DNA inserts. The dotted lines indicate the region of homology between the two inserts.

Fig. 3 shows restriction maps of pMCL 002 and pMCL009 which are derivatives of pMCL 1302, in which the hatched areas denote *E. coli* DNA, and the thin lines denote *Mucor* DNA.

The invention is further described with reference to the following Examples.

MATERIALS AND METHODS

*Strains*

   *Mucor circinelloides* f. *lusitanicus* CBS 277.49 (syn. *Mucor racemosus* ATCC 1216b), *Studies in Mycology 12*, 1976, pp. 1-40,
*Mucor miehei* CBS 370.65, *Studies in Mycology 17*, 1978, pp. 53-71,
*Mucor miehei* CBS 182.67, *Studies in Mycology 17*, 1978, pp. 53-71,
*Mucor racemosus* No. 50, *Nippon Nogeikagaku Kaishi 55*, 1981, pp. 561-571,
*Mucor rouxii* CBS 416.77, *Biochem. Biophys. Acta 58*, 1962, pp. 102-119,
*Mucor pusillus* IMI 96211, *Appl. Microbiol. 16*, 1968, pp. 1727-1733,
*M. circinelloides* leu-2A, was obtained as described in *J. Gen.* Micro*biol.* 105, 1978, pp. 77-81,
*Streptomyces* sp. No. 6, *J. Microbiol. & Serol. 84*, 1968, pp. 173-182, and *J. Gen. Microbiol. 72*, 1972, pp. 281-290,
*Escherichia coli* HB101, *J. Mol. Biol. 41*, p. 459.

*Media and growth conditions*

| | |
|---|---|
| Complete medium: | YPG (3g of yeast extract, 10g of peptone and 20g of glucose per litre of distilled water). |
| Minimal medium: | YNB (0.5g of Difco yeast nitrogen base without amino acids and ammonium sulphate, 1.5g of ammonium sulphate and 1.5g of glutamic acid per litre of distilled water with 1% of glucose and 1$\mu$g/ml thiamine and niacin added after sterilization). |

   For growth of auxotrophs, the minimal medium was supplemented with different amino acids at a final concentration of 100$\mu$g/ml or with casamino acids at a concentration of 2mg/ml. To produce restricted colony growth, the media were adjusted to pH 3.0 with 1M HCl (added after sterilization). Otherwise, the media were adjusted to pH 4.5 as described above. For solid media, pH 4.5, 20g/ml agar was included. In the case of media with a pH of 3.0, double strength solutions of agar and the other media components were autoclaved separately to avoid acid hydrolysis of the agar.
   Cultures of *M. circinelloides, racemosus* and *rouxii* were incubated at 28°C, and cultures of *M. miehei* and *pusillus* were grown at 40°C.
   E. coli was grown at 37°C in LB medium (*Virology 1*, 1955, pp. 190-206) containing 50$\mu$g/ml ampicillin or 25$\mu$g/ml tetracycline as required.

*Chemicals*

   N-glycosyl-polifungin was prepared as described in German Patent No. 2239891. Chitin and chitosan were of practical grade prepared from crab shells. Restriction enzymes, calf alkaline phosphatase, T4 DNA ligase and *E. coli* DNA polymerase I were obtained from Boehringer Mannheim, Germany). Novozym 234 (prepared from *Trichoderma harzianum*, mainly containing $\alpha$-1,3-glucanase activity was obtained from Novo Industries A/S, Denmark), helicase (l'Industrie Biologique, France), laminarinase ($\beta$-1,3-glucanase ex mollusca, Calbiochem, USA), $\beta$-glucanase isolated from *Penicillium emersonii* (BDH Biochemicals, England) and chitinase (Sigma, activity 3.0 units/mg$^{-1}$). All enzymes were used according to the conditions specified by the manufacturers. Polyethylene glycol (PEG) 4000 was from Fluka AG (Switzerland). Heparin, bovine serum albumin (essentially free from fatty acids), 3-(N-morpholino)propane sulphonic acid (MOPS), and RNase A were from Sigma, USA, Sephadex® G50 was obtained from Pharmacia, Sweden, nitrocellulose filters (BA 85) were from Schleicher & Schuell, Federal Republic of Germany, $\alpha^{32}$p-dATP (>600 Ci/mmol) was from New England Nuclear, USA.

*Isolation and assay of Streptozyme*

   Streptomyces sp. No. 6 was grown for 7-8 days at room temperature in the medium according to Skujins et al., *Arch. Biochem. Biophys. 111*, 1965, pp. 358-364, with 0.5% (w/v) chitin and 0.1% (w/v) reprecipitated chitosan (chitosan dissolved in 1% of acetic acid, neutralized with NaOH, then washed extensively with $H_2O$) as the sole carbon sources. Cells were removed by centrifugation (5000 x g for 30 minutes), and the supernatant brought to 95% saturation with ammonium sulphate at 4°C and left overnight with stirring. The precipitate was collected by centrifugation at 5000 x g for 60 minutes, resuspended in a minimal volume of cold 0.02 M sodium phosphate buffer at pH 6.5, and dialyzed extensively against the same buffer at 4°C. After centrifugation to remove insoluble material, the preparation was lyophilized and stored at -20°C. Protein was determined as described in *Anal. Biochem. 72*, 1976, pp. 248-254, using a Bio-Rad protein assay kit I with hen egg albumin as the standard. Chitosanase activity was determined as described in *J. Bacteriol. 124*, 1975, pp. 1574-1585. Chitosan was dissolved in 0.05 M maleic acid to 2 mg/ml, diluted 2-fold with $H_2O$ and the pH adjusted to 6.0 with KOH. After preincubation of 375 $\mu$l aliquots at 30°C for 15 minutes, the reaction was started by addition of 125 $\mu$l enzyme in 0.01 M sodium phosphate buffer, pH 6.5, giving a final volume of 500 $\mu$l containing 0.5 mg/ml chitosan. After a further 15 minutes at 30°C, the reaction was stopped by adding 100 $\mu$l 1 M KOH, and the chitosan precipitated by incubation on ice for 30 minutes. The supernatant obtained after centrifugation in an Eppendorf minifuge for 10 minutes was assayed for hexosamine content by the indole method described in *Methods of Biochemical Analysis*, ed. D. Glick, Interscience Publishers Inc., New York, 1955, vol. 2, pp. 313-358. One unit of chitosanase activity was defined as the activity required to produce 1 $\mu$mol of hexosamine equivalent (mono- or oligosaccharide) per minute.

*Isolation of DNA*

Preparation of plasmid DNA from small cultures of *E. coli* were obtained by the rapid boiling method described in *Anal. Biochem. 114*, 1981, pp. 193-197. They were treated with RNase A (1 mg/ml, preheated at 90°C for 10 minutes) for one hour at 37°C prior to analysis by restriction enzyme digestion and agarose gel electrophoresis. The preparation of plasmids from larger cultures of *E. coli* followed their amplification overnight in the presence of 150 $\mu$g/ml chloramphenicol and was performed according to the method described in *Biochim. Biophys. Acta 299*, 1973, pp. 516-520, with minor modifications. The supernatant obtained after high speed centrifugation of the cell lysate was used directly for purification of the DNA by CsCl gradient centrifugation ($\rho$ = 1.55, containing 0.75 mg/ml ethidium bromide).

*Transformation of E. coli*

*E. coli* HB101 was transformed to ampicillin resistance after induction of cell competence by the $CaCl_2$ method described in *J. Mol. Biol. 53*, 1970, pp. 150-162.

*Hybridization analysis*

DNA fragments generated after restriction endonuclease treatment were electrophoretically separated on agarose gels, then transferred to nitrocellulose filters according to the method described in *J. Mol. Biol. 98*, 1975, pp. 503-517. Plasmid DNA was labelled in vitro with $\alpha^{32}$P-dATP by nick translation according to the method described in *J. Mol. Biol. 113*, 1977, pp. 237-251. Specific radioactivities of 3-4x10$^7$ cpm per $\mu$g DNA were obtained. Hybridization of $^{32}$P-labelled plasmid DNA to the DNA immobilized on nitrocellulose was performed at 68°C in 6 x SSC according to the method described in *Molecular Cloning, a Laboratory Manual*, Cold Spring Harbor, New York, 1982. Autoradiography was carried out using Kodak X-Omat RP X-ray film with Kodak X-Omatic regular intensifying screens at -80°C.

EXAMPLE 1

Isolation of auxotrophic mutants of *Mucor circinelloides* f. *lusitanicus* CBS 277.49

About 10$^8$ spores from the wild type strain CBS 277.49 were plated on YNB medium supplemented with 2 mg/ml casamino acids. The spores were irradiated with a dose of ultraviolet light to give a survival of 0.5-2% and incubated to allow them to complete a full vegetative cycle. For restricted colony growth the medium was adjusted to pH 3.0 with 1 M HCl, and 20 g/l of agar was added. After seven days at 28°C, the next generation of spores produced on each plate were independently collected and kept frozen at -20°C until further use. Survival titres were determined from colony counts of diluted samples before and after the mutagenic treatment.

Cultures inoculated with 10$^8$ spores were grown in 25 ml of liquid minimal medium, and incubated with shaking at 28°C for several hours until about 90% of the spore population had started to germinate (determined by visualisation under the microscope). The germlings were collected by centrifugation, resuspended in an equal volume of YNB without ammonium sulphate, and dispensed into tubes before adding different aliquots of an aqueous solution of N-glycosyl-polifungin. After incubation in the presence of the antibiotic for three hours, the germlings were washed three times with $H_2O$, plated on YNB medium at a dilution yielding 50-100 colonies per plate and incubated for three to five days for colony counting and replica plating.

The colonies surviving the antibiotic treatment were allowed to sporulate and were replicated on plates containing YNB as well as on YNB plates containing casamino acids, to detect auxotrophic mutants. Those colonies which failed to grow on selective minimal medium (YNB) but capable of growing in the presence of casamino acids were classified as auxotrophic mutants and their phenotype was further characterized. These auxotrophs were transferred to various supplemented minimal media, each one of them containing a combination of four or five different amino acids and one including them all (R. Holliday, *Nature 178*, 1956, pp. 987). The nutritional requirement of a particular mutant was identified by detection of growth on one or more of the above-defined combinations.

| Mutagen | Concentration of N-glycosyl -polifungin units/ml | Number of colonies screened | Number of auxotrophs obtained | % Mutants |
|---|---|---|---|---|
| Ultraviolet light | 0 | 952 | 0 | <0,10 |
| " | 1000 | 478 | 21 | 4,39 |

Types of auxotrophs obtained were: 2 requiring leucine, 9 requiring isoleucine, 2 requiring methionine, 1 requiring proline, 1 requiring cysteine or methionine.

The leucine auxotroph R7B, a derivative of *M. circinelloides* CBS 277.49 is deposited at the CBS under the Accession number CBS 753.84.

EXAMPLE 2

Isolation of auxotrophic mutants of *Mucor miehei* CBS 370.65.
The same procedure as under Example 1 was used except that the cultures were incubated at 40° C.

| Mutagen | Concentration of N-glycosyl -polifungin units/ml | Number of colonies screened | Number of auxotrophs obtained | % Mutants |
|---|---|---|---|---|
| None | 1000 | 2521 | 0 | <0,04 |
| Ultraviolet | 0 | 1428 | 2 | 0,14 |
| " | 50 | 867 | 0 | <0,11 |
| " | 100 | 1736 | 0 | <0,06 |
| " | 1000 | 2726 | 10 | 0,36 |

The 12 auxotrophs obtained required lysine.

EXAMPLE 3

*Formation of Protoplasts from M. circinelloides f.lusitanicus*

Sporangiospores from cultures of *M. circinelloides* f.lusitanicus grown for 4-6 days at room temperature on YPG, pH 4.5, agar plates were harvested by gently scraping with a glass rod into distilled water. After one wash in distilled water, they were resuspended in YPG, pH 4.5, at $10^7$/ml and germinated at 28°C with shaking. Ungerminated spores were removed by filtration through nylon cloth, mesh size 16 $\mu$m, and the germlings were washed twice with 0.5 M sorbitol. After resuspension in 0.5 M sorbitol and 0.01 M sodium phosphate buffer, pH 6.5, the germlings were added to an equal volume of streptozyme (cf. MATERIALS AND METHODS) dissolved in the same buffer to give final concentrations of $10^7$/ml germlings and 0.5-1.0 mg/ml streptozyme (corresponding to approximately 1.2 units/ml chitosanase). After incubation at 23°C for up to 4 hours with gentle intermittent mixing, the number of protoplasts produced per ml was calculated by counting a minimum of two samples in a haemocytometer using phase contrast microscopy. When necessary, any undigested hyphae were removed by filtration of the protoplast suspension through nylon cloth, mesh size 10 $\mu$m.

Treatment with streptozyme causes digestion of the cell wall of the germlings, the cell membrane bulges out, is pinched off, and spherical, osmotically sensitive protoplasts with an average size of 7 $\mu$m are released. The formation of protoplasts preferentially occurs at the hyphal apex which is the major site of cell wall synthesis. The total number of protoplasts formed varied with the conditions used, but it could be three times the total number of germlings treated. *Mucor* is coenocytic (i.e. has nonseptate hyphae), and the formation of more than one protoplast per germling thus indicates an effective resealing of the cell membrane after a protoplast has been released.

The thus formed protoplasts of *M. circinelloide*s f. lusitanicus were stable and regenerated at frequencies of up to 40% even after overnight incubation at 4°C in 0.01 M sodium phosphate buffer, pH 6.5, 0.5 M sorbitol. A comparison of different plating conditions for the regeneration of these protoplasts on solid media (2% (w/v) agar) showed that a 1% agar overlay consistently gave better results.

EXAMPLE 4

*Formation of Protoplasts from Mucor miehei*

The experiment was performed as described in Example 3 with the exception that the *M. miehei* cultures were grown at 40°C, and the resulting sporangiospores germinated at 25°C with a 2 hour preincubation at 40°C.

EXAMPLE 5

*Construction of a genomic library of Mucor circinelloides CBS 277.49*

The wild-type *Mucor* DNA used for construction of the genomic library was prepared as follows: Sporangiospores were inoculated into one litre of YPG, pH 4.5, at a density of $10^5$/ml and grown for 16 hours at 28°C with shaking. The mycelium was harvested by filtration through nylon cloth (Monodur, mesh size 22 $\mu$m), washed with cold distilled water, frozen in liquid nitrogen and ground using a mortar and pestle. The frozen powder was warmed to 0°C, an equal volume of TE buffer (10mM-Tris, 1mM-EDTA), pH 8.2, was added, and the slurry was filtered through nylon cloth (Monodur, mesh size 10 $\mu$m). The solid residue was refrozen, reground, warmed to 0°C and combined with the filtrate. The final slurry was brought to 1.5% (w/v) SDS (sodium dodecyl sulphate) and combined with an equal volume of a mixture of phenol (containing 0.1% (w/v) 8-hydroxy-quinoline and saturated with TE buffer, pH 8.2), chloroform and isoamyl alcohol (in the ratio 25:24:1). After shaking at room temperature for five hours, the aqueous phase was obtained by centrifugation (12000 x g, for 10 minutes at room temperature), extracted twice with an equal volume of chloroform and isoamyl alcohol (24:1), and then purified by CsCl gradient centrifugation.

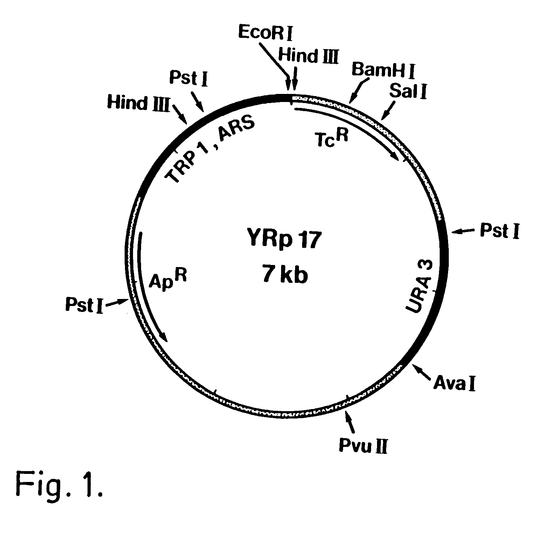

The plasmid used was the yeast-*E.coli* shuttle vector YRp17 (Fig. 1) (D. Botstein, and R.W. Davis, *In the Molecular Biology of the Yeast Saccharomyces cerevisiae*, Cold Spring Harbor, New York, 1982, Vol. IIB, pp. 607). It carries the *TRP1* and *URA3* genes for selection in yeast, and genes conferring resistance to ampicillin and tetracycline in *E. coli*. YRp17 was linearised with *Bam*HI, treated with calf alkaline phosphatase to prevent recircularisation, and then mixed with an equal amount of *Mucor* DNA that had been partially digested with *Mbo*I to produce an average fragment size of 10kb. After treatment with T4 DNA ligase for 16 hours at 12°C, the resulting ligation mixture was used to transform *E. coli* HB101 to ampicillin resistance (M. Mandel and A. Higa, *J. Mol. Biol. 53*, 1970, pp. 159). Of the transformants obtained, 93% were tetracycline sensitive indicating that their plasmids contained *Mucor* DNA inserts. A total of 60.000 transformants were divided into groups containing approximately 2.000 colonies each, amplified overnight by growth in one litre media, and used to prepare 30 independent pools of recombinant plasmid DNA. Aliquots of the plasmid pools obtained were digested with *Bam*HI and electrophoresed in agarose gels (0,7% (w/v)) in order to assess the size of the *Mucor* DNA inserts.

EXAMPLE 6

*Transformation of Mucor circinelloides leucine auxotroph R7B = CBS 753.84*

Sporangiospores of *M. circinelloides* R7B were suspended in 150 ml YPG, pH 4.5, at a concentration of $10^7$/ml and germinated for 3-5 hours at 28°C with shaking. The germlings were harvested by filtration through nylon cloth (Monodur, mesh size 22 $\mu$m), washed extensively with 0,01 M sodium phosphate buffer, pH 6.5, and incubated in the same buffer containing 0.5 M sorbitol, 1.5 mg/ml Novozym 234 and 0.5 mg/ml streptozyme (Skujins et al., *Arch. Biochem. Biophys. 111*, 1965, pp. 358) for 2-3 hours at 23°C as described in Example 3. The protoplasts and any undigested cells remaining were pelleted by centrifugation (400 x g for 5 minutes at room temperature), washed twice with 0.5 M sorbitol, once with 0.5 M sorbitol in MOPS buffer (10 mM 3-(N-Morpholino) propane sulphonic acid, pH 6.3, 50 mM $CaCl_2$), then resuspended in the same solution to give a final volume of 2 ml.

Aliquots of 0.2 ml were added to 20 $\mu$l of the plasmid DNA obtained in Example 5 (0.1-50 $\mu$g, pretreated with 1 mg heparin in 0.5 M sorbitol, MOPS buffer for 20 minutes on ice) plus 20 $\mu$l 40% (w/v) polyethylene glycol (PEG) 4000 in MOPS buffer. After incubation on ice for 30 minutes, 2.5 ml 40% (w/v) PEG 4000 in MOPS buffer was added and the incubation continued at room temperature for 25 min. The suspensions were diluted with 20 ml 0.5 M sorbitol in MOPS buffer, centrifuged (400 x g, 5 minutes at room temperature) and the pellet resuspended in 5 ml YPG pH 4.5, 0.5 M sorbitol. After 30 minutes incubation at room temperature, the cells were centrifuged (400 x g for 5 minutes at room temperature), and then resuspended in 5 ml of YNB, pH 4.5, 0.5 M sorbitol, and 1 ml aliquots were plated in soft agar overlays (9 ml of YNB, pH 3.0, 0.5 M sorbitol, 1% (w/v) agar) onto YNB pH 3.0, 0.5 M sorbitol agar plates. Assessment of the number of colony forming units was made by plating diluted aliquots onto YPG, pH 3.0, 0.5 M sorbitol. Plates were incubated at room temperature for 2-3 days before colonies were counted.

A total of approximately $2 \times 10^6$ viable protoplasts of strain R7B were incubated with 10-50 $\mu$g of recombinant plasmid DNA in the presence of PEG and $CaCl_2$ as described above, and then plated onto minimal media. After two days at room temperature prototrophic colonies appeared which were capable of sporulation and which could be transferred to fresh minimal media with continued growth. No prototrophic colonies were obtained in parallel transformation experiments where either the DNA was omitted, or replaced by 25 $\mu$g of YRp17. Out of the 10 different recombinant plasmid pools tested, a total of four gave rise to Leu[+] colonies. The number of colonies obtained varied from pool to pool but was in the range of 1-38. The fact that the Leu[+] phenotype was due to plasmid-mediated complementation of the original mutation and not to spontaneous reversion was shown by the recovery of recombinant plasmids from the DNA of *Mucor* transformants as described in the following Example.

EXAMPLE 7

*Transformation of E. coli with recombinant plasmids obtained from Mucor transformants*

DNA from small cultures of *Mucor* for the analysis of transformants was obtained substantially as described in Yelton et al., *Proc. Natl. Acad. Sci. USA 84*, 1984, pp. 1470. Cultures were grown from a spore inoculum in 100 ml of YNB, pH 4.5, for 20 hours at 28°C, after which the mycelium was harvested and ground as described above in Example 5. The ground cells were suspended in 10 ml 50 mM EDTA, pH 8.5, 0.2% (w/v) SDS, and mixed for one minute at room temperature. The lysate was heated at 68°C for 15 minutes, cooled to room temperature and centrifuged (12000 x g for 15 minutes at room temperature). The supernatant (10 ml) was cooled on ice, 0.6 ml of 8.0 M potassium acetate, pH 4.2, was added, and the mixture incubated on ice for 60 minutes. After centrifugation (25000 x g, 15 minutes at 4°C), the nucleic acids present in the supernatant were precipitated at room temperature by the addition of an equal volume of isopropanol and collected by centrifugation (12000 x g for 30 minutes at room temperature). The pellet was resuspended in 6.0 ml of TE buffer, pH 7.6, and treated with 60 $\mu$l RNase A (10 mg/ml, preheated at 90°C for 10 minutes) for 1 hour at 37°C. After extraction with an equal volume of phenol, nucleic acids were precipitated at 20°C after the addition of 0.1 volumes 3.0 M sodium acetate, pH 4.5, and 2.5 volumes ethanol. The pellet obtained after centrifugation (12000 x g for 30 minutes at 4°C) was resuspended in 500 $\mu$l TE buffer, pH 8.0, and the DNA content estimated by agarose gel electrophoresis.

Total DNA from 19 different Leu[+] *Mucor* transformants (cf. Example 6) grown in minimal medium was treated with *Kpn*I, self-ligated and used to transform *E. coli* HB101 (cf. MATERIALS AND METHODS). One ampicillin resistant colony was obtained. The plasmid recovered from this transformant was designated pMCL1302. The strain of *E. coli* HB101 pMCL1302 is deposited in the Laboratory of Microbiology at Delft (LMD) under the Accession No. LMD 84.94.

In order to increase the number of bacterial transformants obtained, the *Mucor* DNA was purified by CsCl gradient centrifugation. DNA from five Leu[+] transformants was then successfully digested with *Sal*I, self-ligated and used to transform *E. coli*. In this way, four ampicillin resistant colonies were obtained from two different DNA preparations. The plasmid recovered from one of these was designated pMCL1647. The strain of *E. coli* HB101 pMCL1647 is deposited in the LMD under the Accession No. LMD 84.95. Plasmid DNA from small cultures of *E. coli* as well as larger cultures of *E. coli* was prepared as described in MATERIALS AND METHODS.

Physical maps of plasmids pMCL1302 and pMCL1647 were obtained by single and double digestions with seven restriction enzymes (Fig. 2). They consist of YRp17 with *Mucor* DNA inserts of 10.5 kb and 4.0 kb respectively. The inserts in the two plasmids have a homologous region of 3.7 kb delimited by two *Ava*I sites, but placed in the opposite orientation relative to the YRp17 sequences. They originate from *Mucor* colonies transformed to Leu[+] with recombinant plasmid DNA from pools No. 13 and No. 16, respectively.

Plasmid pMCL1647 is smaller than the original transforming plasmid from pool No. 16, the insert of which can be estimated to be at least 9.9 kb.

EXAMPLE 8

*Transformation of M. circinelloides R7B*

The transformation was performed as described in Example 6.

The frequency of transformation of strain R7B to Leu[+] using the plasmid pMCL1302 is shown in the following table:

| Experiment | DNA µg | No. viable cells plated | No. Leu+ colonies | transformation frequency | |
|---|---|---|---|---|---|
| | | | | per µg DNA | per viable cell |
| 1 | 0 | $2.9 \times 10^7$ | 0 | 0 | 0 |
| | 0.1 | $2.9 \times 10^7$ | 72 | 720 | $2.5 \times 10^{-6}$ |
| | 0.5 | $2.9 \times 10^7$ | 120 | 240 | $4.1 \times 10^{-6}$ |
| | 1.0 | $2.9 \times 10^7$ | 216 | 216 | $7.4 \times 10^{-6}$ |
| 2 | 0 | $3.2 \times 10^6$ | 0 | 0 | 0 |
| | 1.0 | $3.2 \times 10^6$ | 600 | 600 | $1.9 \times 10^{-4}$ |
| 3 | 0 | $5 \times 10^5$ | 0 | 0 | 0 |
| | 1.0 | $5 \times 10^5$ | 193 | 193 | $3.9 \times 10^{-4}$ |
| | 5.0 | $5 \times 10^5$ | 234 | 47 | $4.7 \times 10^{-4}$ |
| | 25.0 | $5 \times 10^5$ | 473 | 19 | $9.5 \times 10^{-4}$ |

Both the concentration of DNA and the density of cells plated were found to have an influence on the number of Leu[+] colonies obtained. Up to 720 transformants per µg DNA were obtained using 0.1-1.0 µg plasmid DNA. An increase in the amount of DNA used gave an increase in the number of transformants but the frequency of transformation (per µg DNA) progressively decreased. This indicates that the DNA concentration was not the sole limiting factor. In terms of the number of viable cells plated the transformation frequency varied from $2.5 \times 10^{-6}$ to $8 \times 10^{-4}$. The relatively low frequency obtained at the highest plating density probably reflects the inhibition of growth of the transformed cells by the non-transformed cells.

Treatment of strain R7B with pMCL 1647 also gave rise to Leu⁺ colonies but the frequency of transformation was much lower than that obtained using pMCL 1302. In an experiment conducted concurrently with experiment 1, above, 1.0 µg and 5.0 µg pMCL 1647 gave rise to 6 and 17 stable Leu⁺ colonies, respectively.

These results demonstrate that the two plasmids pMCL 1302 and pMCL 1647 can serve as *Mucor-yeast-E. coli* shuttle vectors and can be used to obtain high frequency genetic transformation of *Mucor* with recombinant DNA.

Both vectors have a unique *Sal*I restriction site (Fig. 2) which may be used for inserting a DNA sequence coding for a desired gene product. Cloning into this site does not affect the DNA sequences required for the expression of the yeast genes TRP1 and URA3 and for autonomous replication of the vector (ARS). Nor does cloning of a DNA sequence for the desired product into this site interfere with the *Mucor* gene coding for leucine prototrophy and the *E. coli* ampicillin resistance gene, thus permitting maintenance and selection in all three hosts.

*M. circinelloides* R7B-1302 carrying the plasmid pMCL 1302 is deposited in the CBS under the Accession number CBS 754.84.

*M. circinelloides* R7B-1647 carrying the plasmid pMCL 1647 is deposited in the CBS under the Accession number CBS 755.84.

EXAMPLE 9

*Hybridization analysis of Leu⁺ Mucor transformants*

Final confirmation that the Leu⁺ colonies obtained in Example 6 were transformants was provided by the detection of YRp17 plasmid sequences within their DNA. Total DNA was isolated from a number of transformants and purified by CsCl gradient centrifugation. The fragments obtained after digestion with *Bam*HI or *Pst*I were separated by agarose gel (0.7% w/v) electrophoresis, transferred to nitrocellulose filters (E.M. Southern, *J. Mol. Biol. 98*, 1975, pp. 503) and hybridized to ³²P-labelled (P.W.J. Rigby et al., *J. Mol. Biol. 113*, 1977, p. 237) YRp17. Hybridization was performed at 68°C in 6 x SSC (0.15 M NaCl, 0.015 M trisodium citrate, pH 7.0) according to Maniatis et al. (Cold Spring Harbor, New York, 1982). Autoradiography was carried out using Kodak X-Omat RP X-ray film with Kodak X-Omatic regular intensifying screens at -80°C.

All 22 transformants examined showed specific hybridization to YRp17, while DNA isolated from the recipient strain R7B contained no sequences complementary to this probe. The hybridization patterns obtained were of two types and are presented in the following table.

| To labelled YRp17 hybridise: | | | | | | | | |
|---|---|---|---|---|---|---|---|---|
| DNA fragments in kilobases from | YRp17 | | R7B | | transformant R7B-1302 | | transformant R7B-1611 | |
| after digestion with: | *Bam*HI | *Pst*I | *Bam*HI | *Pst*I | *Bam*HI | *Pst*I | *Bam*HI | *Pst*I |
| | 7 | 3.2 | - | - | 12 | 4.7 | 11 | 7.0 |
| | | 2.3 | | | | 3.2 | | 5.2 |
| | | | | | | | | 3.2 |
| | | | | | | 2.0 | | |
| | | 1.5 | | | | 1.5 | | 1.5 |

The patterns given for transformant 1302 are representative of all five transformants examined which originate from plasmid pool No. 13. Similarly, those given for 1611 are representative of all 14 transformants examined which originate from plasmid pool No. 16. While the 3.2 kb and 1.5 kb *Pst*I fragments of YRp17 are present in all transformants, the 2.3 kb *Pst*I fragment which contained the *Bam*HI cloning site has been replaced by two, more weakly hybridising fragments, indicating the presence of an insert with at least one internal *Pst*I site. After *Bam*HI digestion a single hybridising fragment is obtained in all transformants. These fragments are larger than linear YRp17 indicating the presence of inserts within the plasmid DNA.

The hybridisation of $^{32}$P-labelled plasmid pMCL 1302 to *Bam*HI and *Pst*I digests of DNA from the untransformed strain R7B and from the Leu[+] transformant 1302 reveals that the plasmid contains inserts of *Mucor* DNA:

To labelled pMCL 1302 hybridize after digestion:

DNA fragments in kilobases from:

| | •with *Bam*HI | | | with *Pst*I | | |
|---|---|---|---|---|---|---|
| | pMCL1302 | R7B | trans-formant R7B1302 | pMCL1302 | R7B | trans-formant R7B1302 |
| | 12 | 16 | 12 | 2x4.4 | *8.2* | *8.2* |
| | 2.9 | 14 | *5.8* | 3.2 | *5.2* | *5.2* |
| | 2.1 | *5.8* | *3.3* | 2.2 | *4.4* 3x | *4.4* |
| | | *3.3* | 2.9 | 1.6 | | 3.2 |
| | | 2.0 | 2.2 | 0.95 | | 2.2 |
| | | | | 0.7 | | 1.6 |
| | | | | | | 0.95 |
| | | | | | | 0.7 |

Since the DNA from strain R7B shares no homology with YRp17, the fragments hybridising from R7B represent *Mucor* genomic DNA present only in the insert of pMCL 1302. The hybridisation of pMCL 1302 to DNA from the Leu[+] transformant 1302 reveals hybridisation to both sequences derived from the YRp17 plasmid and the genomic sequences present in strain R7B. The homologous fragments have been italicized in the Tables shown above.

This proves that the plasmids pMCL 1302 and, analogously, pMCL 1647 are plasmids which contain *Mucor, E. coli* and yeast genes.

EXAMPLE 10

*Autonomous replication in Mucor*

In order to detect the presence of free plasmid molecules in *Mucor* transformants, DNA isolated from the transformants was electrophoretically separated on 0.4% (w/v) agarose gels, with or without prior treatment with restriction endonucleases and then transferred to nitrocellulose filters according to the method described by Southern.

To the labelled 3.7 kb *Aval* fragment
of the pMCL1302 plasmid hybridize:

| with undigested DNA Rf in mm | | | after digestion with *Hpal*, fragments in kilobases and plasmids of relative mobility in Rf | |
|---|---|---|---|---|
| transformant | | | | transformant |
| R7B | R7B 1302 | pMCL1302 | R7B | R7B 1302 |
| smear | 6 mm | 7 mm | ~25 kb | 6 mm |
| typical | 14 mm | 14 mm | | 14 mm |
| for chro- | chromosomal | | | |
| mosomal | band | | | ~25 kb |
| DNA | 29 mm | 21 mm | | 29 mm |
| | 36 mm | | | 36 mm |
| | 46 mm | 46 mm | | 46 mm |

When the total DNA isolated from *Mucor* transformants is electrophoresed in a 0.4% (w/v) agarose gel and hybridised to the $^{32}$P-labelled 3.7 kb *Aval* fragment of pMCL 1302, unique hybridization bands can be seen in the undigested DNA of the transformant but not in that of the Leu- strain as shown in the table above. The electrophoretic mobility of these bands is inversely proportional to the size of the transforming plasmid and the lowest band comigrates with covalently closed plasmid circles isolated from *E. coli*. In order to confirm that the hybridization bands represent free plasmid molecules, the DNA was treated with the restriction endonuclease *Hpal* for which there are cleavage sites in the genomic DNA, but not in pMCL 1302. Any sequences integrated into the genomic DNA should have altered the electrophoretic mobility after *Hpal* digestion, while free plasmid molecules should remain unaffected. Digestion of the genomic DNA of strain R7B results in the appearance of a unique band (~25 kb) of hybridization to the 3.9 kb *Aval* probe. Digestion of the transformant DNA does not otherwise alter the hybridization pattern obtained. Equivalent results were obtained with strain R7B transformed with the plasmid pMCL 1647.

In order to further verify the presence of autonomously replicating plasmids, these were recovered from isolated uncut *Mucor* DNA. After transformation of *M. circinelloides* R7B to Leu$^+$ with plasmids pMCL 1302 or pMCL 1647, DNA was isolated from transformants as described in Example 7 and then used directly to transform *E. coli* HB101. The number of amp$^r$ colonies obtained ranged from 0-4 per µg DNA, with up to 200 totally from the DNA extracted from 100 ml of culture. Restriction fragment analysis of the plasmids isolated showed 35 to be identical to the original transforming plasmids.

The mitotic stability of the autonomously replicating plasmids was tested as follows: *M. circinelloides* R7B was transformed to Leu$^+$ with pMCL 1302, and then three transformants were transferred to minimal and complete medium and allowed to complete the full vegetative growth cycle culminating in sporangiospore formation. The percentage of viable sporangiospores retaining the Leu$^+$ phenotype was estimated by plating sporangiospore suspensions onto complete medium followed by replica plating of colonies onto minimal medium and/or parallel plating of dilutions onto complete and minimal medium. Only 5% of viable sporangiospores were Leu$^+$ after the first transfer to complete medium compared with 45% when growth was continued under selective conditions. A further two successive transfers to complete medium reduced the percentage of Leu$^+$ sporangiospores to 0.8% and 0.056%, respectively.

To estimate plasmid copy number in strain R7B transformed with pMCL 1302, total *Mucor* DNA from strains R7B and R7B 1302 was digested with *SalI*, denatured in 0.4 M NaOH for 10 minutes and chilled and diluted with an equal volume of cold 2 M ammonium acetate. Two-fold serial dilutions were applied in 100 µl aliquots to a nitrocellulose filter using a Schleicher & Schuell filtration manifold. The filter was washed in 1 M ammonium acetate, then 2 × SSC, then 2 × Denhardt's solution before baking at 80°C for two hours.

Hybridization of the DNA on this filter to the 3.7 kb *Aval* fragment of *Mucor* DNA indicates that the plasmid molecules are present in the transformants at 4-5 copies per haploid genome.

The data presented above is proof of the autonomous replication in *Mucor* of hybrid plasmids constructed from fragments of *Mucor* DNA inserted in YRp17. These plasmids transform *Mucor* at very high frequencies and can be recovered in an unmodified form from the transformant DNA. They are efficient shuttle vectors for the transfer of genetic material between *Mucor, E. coli* and *S. cerevisiae* and enable an easy recovery of *Mucor* genes cloned by direct complementation from a genomic library.

EXAMPLE 11

*Identification of an autonomous replication sequence of Mucor*

Plasmid pMCL 1302 (Fig. 2) contains the sequences denoted ARS and ORI which are responsible for the maintenance of the plasmid as an autonomously replicating unit in *Saccharomyces cerevisiae* and *Escherichia coli*, respectively. In order to determine their role, if any, in the autonomous replication of pMCL 1302 within *Mucor*, subclones lacking these sequences were prepared and used to transform *M. circinelloides* R7B to Leu[+].

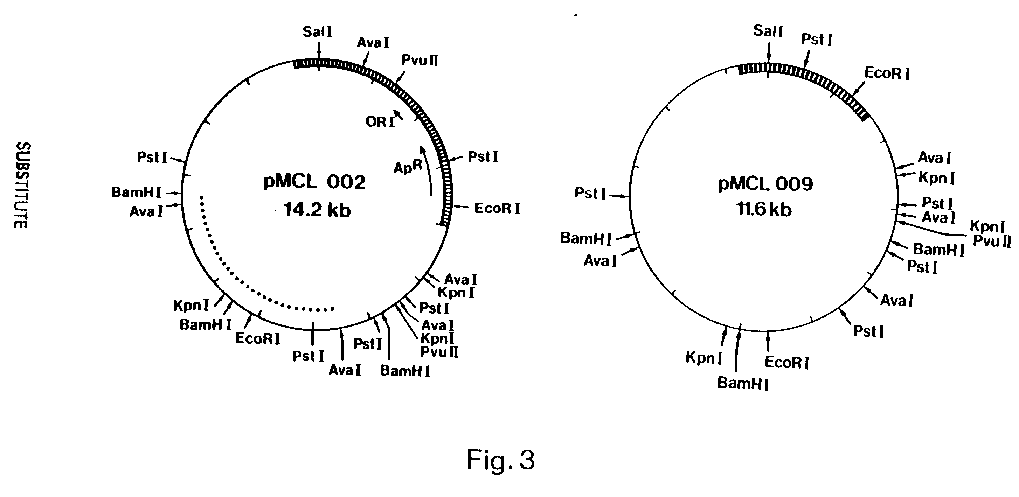

Plasmid pMCL 002 (Fig. 3) consists of the *Mucor* DNA insert of pMCL 1302 cloned into pBR322. It is devoid of all yeast sequences present in pMCL 1302, but is still found to exist as a free plasmid within *Mucor* transformants.

Plasmid pMCL 009 (Fig. 3) was prepared by digestion of pMCL 002 with *Bgl*I which cleaves at three positions within the pBR322 sequence, but not at all within the insert of *Mucor* DNA. The largest of the resulting restriction fragments contains the entire *Mucor* DNA insert plus 1.8 kb of pBR322 and lacks the *E. coli* ORI plus at least 1 kb of pBR322 DNA on either side of this region. After isolation by agarose gel electrophoresis, this fragment was treated with T4-DNA ligase, and then the ligation mix was used directly to transform *M. circinelloides* R7B to Leu[+]. DNA was isolated from four transformants and analyzed by Southern hybridization to $^{32}$P-labelled pMCL 002. The uncut isolated DNA of these transformants gave hybridization patterns indicating the presence of free plasmid molecules. Digestion with *Ava*I results in a pattern of hybridization consistent with the presence of pMCL 009, not pMCL 002, within the transformants. The absence of the *E. coli* ORI region is indicated by the replacement of the 4.1 and 4.7 kb *Ava*I fragments of pMCL 002 by a single 6.5 kb fragment.

BIBLIOGRAPHY

M.E. Case, M. Schweizer, S.R. Kushner & N.H. Giles, "Efficient transformation of *Neurospora crassa* by utilizing hybrid plasmid DNA", *Proc. Natl. Acad. Sci. USA 76*, 1979, pp. 5259-5263.

U. Stahl, P. Tudzynski, U. Kück & K. Esser, "Replication and expression of a bacterial-mitochondrial hybrid plasmid in the fungus. *Podospora anserina*", *Proc. Natl. Acad. Sci. USA 79*, 1982, pp. 3641-3645.

D.J. Ballance, F.P. Buxton & G. Turner, "Transformation of *Aspergillus nidulans* by the orotidine-5'-phosphate decarboxylase gene of *Neurospora crassa*", *Biochem. Biophys. Res. Common. 112*, 1983, pp. 284-289.

M. Sternberg, "Microbial Rennets", *Advances in Applied Microbiology 20*, 1976, pp. 135-157.

K. Aunstrup, "Proteinases", in A.H. Rose, ed., *Microbial Enzymes & Bioconversions; Economic Microbiology*, Vol. 5, Academic Press, New York, 1980, pp. 50-115.

US Patent No. 3,988,207.

K. Aunstrup, Production, Isolation and Economics of Extracellular Enzymes", in: L.B. Wingard, E. Katchalski-Katzir and L. Goldstein, eds., *Applied Biochemistry and Bioengineering*, Vol. 2, Academic Press, New York, 1979, pp. 28-69.

W.M. Fogarty, & C.T. Kelly, "Amylases, amyloglucosidases and related glucanases", in A.H. Rose, ed., *Microbial Enzymes & Bioconversions; Economic Microbiology*, Vol. 5, Acad. Press, New York, 1980, pp. 116-170.

K. Nishimori, N. Shimizu, Y. Kawaguchi, M. Hidaka, T. Vozumi & T. Beppu, "Expression of Cloned Calf Procymosin cDNA under Control of the Tryptophan Promoter", *Gene 29*, 1984, pp. 41-49.

J.S. Emtage, S. Angal, M.T. Doel, T.J.R. Harris, B. Jenkins, G. Lilley & P.A. Lowe, "Synthesis of Calf Prochymosin (Prorennin) in *Escherichia coli*", *Proc. Natl. Acad. Sci. USA 80*, 1983, pp. 3671-3675.

J. Mellor, M.J. Dobson, N.A. Roberts, M.F. Tuite, J.S. Emtage, S. White, P.A. Lowe, T. Patel, A.J. Kingsman & S.M. Kingsman, "Efficient Synthesis of Enzymatically Active Calf Chymosin in Saccharo*myces cerevisiae*", *Gene 24*, 1983, pp. 1-14.

C.G. Goff, D.T. Moir, T. Kohno, T.C. Gravius, R.A. Smith, E. Yamasaki & A. Taunton-Rigby, "Expression of Calf Prochymosin in *Saccharomyces cerevisiae*", *Gene 27*, 1984, pp. 35-46.

M.A.A. Schipper, "On *Mucor circinelloides, Mucor racemosus* and Related Species", *Studies in Mycology 12*, 1976, pp. 1-40.

R. Holliday, "A New Method for the Identification of Biochemical Mutants of Micro-organisms", *Nature 178*, 1956, pp. 987.

D. Botstein, & R.W. Davis, "Principles and Practice of Recombinant DNA Research with Yeast", in J.N. Strathern, E.W. Jones and J.R. Broach, eds., *The Molecular Biology of the Yeast Saccharomyces cerevisiae*, Vol. IIB, Cold Spring Harbor, New York, 1982, pp. 607-636.

M. Mandel, & A. Higa, "Calcium-dependent Bacteriophage DNA Infection", *J. Mol. Biol. 53*, 1970, pp. 159-162.

J.J. Skujins, H.J. Potgieter & M. Alexander, "Dissolution of fungal cells by a Streptomycete chitinase and $\beta$ (1-3) glucanase", *Arch. Biochem. Biophys. 111*, 1965, pp. 358-364.

M.M. Yelton, J.E. Hamer & W.E. Timberlake, "Transformation of *Aspergillus nidulans* by Using a trpC Plasmid", *Proc. Natl. Acad. Sci. USA 84*, 1984, pp. 1470-1474.

P.S. Holmes, & M. Quigley, "A Rapid Boiling Method for the Preparation of Bacterial Plasmids", *Anal. Biochem. 114*, 1981, pp. 193-197.

P.S. Gordon, & D. Vapnek, "A simple Method of Preparing Large Amounts of $\phi$X174 RFI Supercoiled DNA", *Biochem. Biophys. Acta 299*, 1973, pp. 516-520.

E.M. Southern, "Detection of Specific Sequences among DNA Fragments Separated by Gel Electrophoresis", *J. Mol. Biol. 98*, 1975, pp. 503-517.

P.W.J. Rigby, M. Dieckmann, C. Rhodes & P. Berg, "Labeling Deoxy-ribonucleic Acid to High Specific Activity in vitro by Nick Translation with DNA Polymerase I", *J. Mol. Biol. 113*, 1977, pp. 237-251.

T. Maniatis, E.F. Fritsch & J. Sambrook, *"Molecular Cloning, a Laboratory Manual*, Cold Spring Harbor, New York, 1982.

## Claims

**Claims for the following Contracting States : BE, CH, LI, DE, FR, GB, IT, LU, NL and SE**

1. A method of transforming a fungus of the class Zygomycetes, in which sporangiospores or germlings of a fungus of the class Zygomycetes are transformed with a recombinant expression vector which is replicated in the fungal species, the transformation being effected by treating germinating sporangiospores with one or more suitable lytic enzymes, transforming the resulting protoplasts with an expression vector, and regenerating the cell wall.

2. A method according to claim 1, in which the fungus is a fungus of the genus *Mucor*.

3. A method according to claim 2, in which the *Mucor* strain is selected from a mesophilic *Mucor* species such as *M. circinelloides, M.* racemosus or *M. rouxii*, or a thermophilic *Mucor* species such as *M. miehei* or *M. pusillus*.

4. A method according to claim 1, in which the lytic enzyme is chitosanase.

5. A method according to claim 1, in which the fungal strain of the class Zygomycetes is a suitable auxotrophic or antibiotic sensitive mutant strain which, when transformed, is complemented by an expression vector carrying the counter-selectible prototrophic or antibiotic resistance gene.

6. A method according to any of the preceding claims, in which the expression vector is self-replicating.

7. A method according to any of claims 1-5, in which the expression vector is integrated stably into one or more of the host chromosomes.

8. A method according to any of the preceding claims in which the vector is a shuttle vector which can be replicated in a fungal species of the class Zygomycetes. *E coli* and/or a yeast.

9. A method according to claim 8, in which the vector is pMCL 1302, carried in *Mucor circinelloides* R7B, and deposited in the Centraal Bureau voor Schimmelkultern under the Accession No. CBS 754.84.

10. A method according to claim 8, in which the vector is pMCL 1647, carried in *Mucor circinelloides* R7B, and deposited in the Centraal Bureau voor Schimmelkulturen under the Accession No. CBS 755.84.

11. A replicable expression vector for transformation of a fungus of the class Zygomycetes comprising

a DNA sequence from a fungus of the class Zygomycetes which comprises a selectable marker,

at least one restriction site for the insertion of a DNA sequence coding for a desired gene product located at a position where the insertion of the DNA sequence does not influence replication or selection of said vector,

and optionally a sequence coding for autonomous replication of the vector in a fungus of the class Zygomycetes,

and being obtainable by the following steps:

1) Preparing a recombinant plasmid, containing a genomic DNA fragment of a member of the class Zygomycetes and being replicable in a bacterium and/or a yeast, in a manner known per se,

2) Transforming a deficient mutant of a member of the class Zygomycetes with said recombinant plasmid by treating germinating sporangiospores with one or more suitable lytic enzymes, transforming the resulting protoplasts with said recombinant plasmid and regenerating the cell wall, and

3) Selecting transformed mutants and isolating said plasmids from same in a manner known per se.

**12.** An expression vector according to claim 11, in which the marker is a gene mediating antibiotic resistance to an antibiotic sensitive fungal strain or a gene mediating prototrophy to an auxotrophic fungal strain.

**13.** An expression vector according to claim 11 or 12 which is self-replicating.

**14.** An expression vector according to claim 11 or 12 which is integrated stably into one or more of the host chromosomes.

**15.** An expression vector according to any of claims 11-14 which is a shuttle vector which can be replicated in a species of the class Zygomycetes, *E. coli* and/or a yeast.

**16.** An expression vector according to claim 15, which is pMCL 1302, carried in *Mucor circinelloides* R7B, and deposited in the Centraal Bureau voor Schimmelkulturen under the Accession No. CBS 754.84.

**17.** An expression vector according to claim 15, which is pMCL 1647, carried in *Mucor circinelloides* R7B, and deposited in the Centraal Bureau voor Schimmelkulturen under the Accession No. CBS 755.84.

**18.** A fungus of the class Zygomycetes, which carries a recombinant expression vector which is replicated in the organism.

**19.** A fungus according to claim 18, in which the expression vector is a vector according to any of claims 31-38 or 11-17.

**20.** A fungus according to claim 18 or 19, which is a fungus of the genus *Mucor.*

**21.** A fungus according to claim 20, which is a *Mucor* strain selected from a mesophilic *Mucor* species such as *M. circinelloides, M. racemosus* or *M. rouxii* or a thermophilic *Mucor* species such as *M. miehei* or *M. pusillys.*

**22.** A fungus according to any of the claims 18-21, which is an auxotrophic mutant strain.

**23.** A method of preparing an expression vector according to any of the claims 31-38, comprising inserting, in a plasmid which is replicated in a fungus of the class Zygomycetes, a DNA sequence for a marker for the identification and/or selection of cells transformed with the vector, and providing a restriction site for the insertion of a DNA sequence coding for a desired gene product at a location where said DNA sequence does not interfere with replication and/or selection, and inserting said DNA sequence for the desired gene product at this site.

**24.** A method of producing a gene product from a fungus of the class Zygomycetes, in which sporangiospores or germlings of a fungus of the class Zygomycetes are transformed with a recombinant expression vector which is replicated in the fungal species and which carries a DNA sequence coding for a desired gene product according to the method of claim 1, the transformed sporangiospores or germlings are grown in a suitable culture medium to express said DNA sequence, and the resulting product is harvested.

**25.** A method according to claim 24, in which the product of the inserted DNA is excreted into the medium and recovered therefrom.

**26.** A method according to claim 24 or 25, in which the gene product produced from the Zygomycetes species is a polypeptide or protein or fragment thereof, an enzyme or a non-proteinaceous product of reactions of enzymes with a compound in the culture medium, or a low molecular weight product such as hormones or nucleic acids.

**27.** A method according to claim 26, in which the gene product is an enzyme.

**28.** A method according to claim 27, in which the enzyme is lipase, amyloglucosidase, $\alpha$-amylase, $\beta$-galactosidase, cellulase or proteases such as chymosin.

**29.** A method according to claim 24, in which the expression vector is a vector according to any of claims 31-38.

**30.** A method according to claim 29, in which the fungus is a fungus of the genus *Mucor*.

**31.** A replicable expression vector according to Claim 11 which further contains in said restriction site a DNA sequence which can be expressed in a fungus of the class Zygomycetes and codes for a desired gene product.

**32.** An expression vector according to claim 31, in which the marker is a gene mediating antibiotic resistance to an antibiotic sensitive fungal strain or a gene mediating prototrophy to an auxotrophic fungal strain.

**33.** An expression vector according to claim 31 or 32 which is self-replicating.

**34.** An expression vector according to claim 31 or 32 which is integrated stably into one or more of the host chromosomes.

**35.** An expression vector according to any of claims 31-34 which is a shuttle vector which can be replicated in a fungal species of the class Zygomycetes, *E. coli* and/or a yeast.

**36.** An expression vector according to any of claims 31-35 in which the gene product encoded by the DNA sequence is a polypeptide or protein or fragment thereof, an enzyme or a non-proteinaceous product of reactions of enzymes with a compound in the culture medium, or a low molecular weight product such as hormones or nucleic acids.

**37.** An expression vector according to claim 36, in which the gene product is an enzyme.

**38.** An expression vector according to claim 37, in which the enzyme is lipase, amyloglucosidase, $\alpha$-amylase, $\beta$-galactosidase, cellulase or protease such as chymosin.

**Claims for the following Contracting State : AT**

**1.** A method of transforming a fungus of the class Zygomycetes, in which sporangiospores or germlings of a fungus of the class Zygomycetes are transformed with a recombinant expression vector which is replicated in the fungal species, the transformation being effected by treating germinating sporangiospores with one or more suitable lytic enzymes, transforming the resulting protoplasts with an expression vector, and regenerating the cell wall.

**2.** A method according to claim 1, in which the fungus is a fungus of the genus *Mucor*.

**3.** A method according to claim 2, in which the *Mucor* strain is selected from a mesophilic *Mucor* species such as *M. circinelloides, M. racemosus* or *M. rouxii*, or a thermophilic *Mucor* species such as *M.*

*miehei* or *M. pusillus*.

4. A method according to claim 1, in which the lytic enzyme is chitosanase.

5. A method according to claim 1, in which the fungal strain of the class Zygomycetes is a suitable auxotrophic or antibiotic sensitive mutant strain which, when transformed, is complemented by an expression vector carrying the counter-selectible prototrophic or antibiotic resistance gene.

6. A method according to any of the preceding claims, in which the expression vector is self-replicating.

7. A method according to any of claims 1-5, in which the expression vector is integrated stably into one or more of the host chromosomes.

8. A method according to any of the preceding claims in which the vector is a shuttle vector which can be replicated in a fungal species of the class Zygomycetes, *E. coli* and/or a yeast

9. A method according to claim 8, in which the vector is pMCL 1302, carried in *Mucor* circinelloides R7B, and deposited in the Centraal Bureau voor Schimmelkulturen under the Accession No. CBS 754.84.

10. A method according to claim 8, in which the vector is pMCL 1647, carried in *Mucor circinelloides* R7B, and deposited in the Centraal Bureau voor Schimmelkulturen under the Accession No. CBS 755.84.

11. A method for preparing an expression vector for the transformation of a fungus of the class Zygomycetes by combining a DNA sequence coding for a desired gene product, and a marker for the identification and/or selection of cells transformed with the vector, the vector being replicated in fungi of the class Zygomycetes.

12. A method according to claim 11, in which the marker is a gene mediating antibiotic resistance to an antibiotic sensitive fungal strain or a gene mediating prototrophy to an auxotrophic fungal strain.

13. A method according to claim 11 or 12 which is self-replicating.

14. A method according to claim 11 or 12 which is integrated stably into one or more of the host chromosomes.

15. A method according to any of claims 11-14 which is a shuttle vector which can be replicated in a fungal species of the class Zygomycetes, *E. coli* and/or a yeast.

16. A method according to any of claim 11-15, in which, the gene product encoded by the DNA sequence is a polypeptide or protein or fragment thereof, an enzyme or a non-proteinaceous product of reactions of enzymes with a compound in the culture medium, or a low molecular weight product such as hormones or nucleic acids.

17. A method according to claim 16, in which the gene product is an enzyme.

18. A method according to claim 17, in which the enzyme is lipase, amyloglucosidase, $\alpha$-amylase, $\beta$-galactosidase, cellulase or protease such as chymosin.

19. A method for preparing an expression vector for the transformation of a fungus of the class Zygomycetes by combining a marker for the identification and/or selection of cells transformed with the vector, and at least one restriction site for the insertion of DNA sequence coding for a desired gene product, the vector being replicated in fungi of the class Zygomycetes.

20. A method according to claim 19, in which the marker is a gene mediating antibiotic resistance to an antibiotic sensitive fungal strain or a gene mediating prototrophy to an auxotrophic fungal strain.

21. A method according to claim 19 or 20 which is self-replicating.

22

**22.** A method according to claim 19 or 20 which is integrated stably into one or more of the host chromosomes.

**23.** A method according to any of claims 19-22 which is a shuttle vector which can be replicated in a species of the class Zygomycetes, *E. coli* and/or a yeast.

**24.** A method according to claim 23, which is pMCL 1302, carried in *Mucor circinelloides* R7B, and deposited in the Centraal Bureau voor Schimmelkulturen under the Accession No. CBS 754.84.

**25.** A method according to claim 23, which is pMCL 1647, carried in *Mucor circinelloides* R7B, and deposited in the Centraal Bureau voor Schimmelkulturen under the Accession No. CBS 755.84.

**26.** A method for preparing a fungus of the class Zygomycetes by introducing a recombinant expression vector which is replicated in the organism.

**27.** A method according to claim 26, in which the expression vector is a vector according to any of claims 11-18 or 19-25.

**28.** A method according to claim 26 or 27, which is a fungus of the genus *Mucor*.

**29.** A method according to claim 28, which is a *Mucor* strain selected from a mesophilic *Mucor* species such as *M. circinelloides*, *M. racemosus* or *M. rouxii* or a thermophilic *Mucor* species such as *M. miehei* or *M. pusillys*.

**30.** A method according to any pf the claims 26-29, which is an auxotrophic mutant strain.

**31.** A method of preparing an expression vector according to any of the claims 11-18, comprising inserting, in a plasmid which is replicated in a fungus of the class Zygomycetes, a DNA sequence for a marker for the identification and/or selection of cells transformed with the vector, and providing a restriction site for the insertion of a DNA sequence coding for a desired gene product at a location where said DNA sequence does not interfere with replication and/or selection, and inserting said DNA sequence for the desired gene product at this site.

**32.** A method of producing a gene product from a fungus of the class Zygomycetes. In which sporangiospores or germlings of a fungus of the class Zygomycetes are transformed with a recombinant expression vector which is replicated in the fungal species and which carries a DNA sequence coding for a desired gene product according to the method of claim 1, the transformed sporangiospores or germlings are grown in a suitable culture medium to express said DNA sequence, and the resulting product is harvested.

**33.** A method according to claim 32, in which the product of the inserted DNA is excreted into the medium and recovered therefrom.

**34.** A method according to claim 32 or 33, in which the gene product produced from the Zygomycetes species is a polypeptide or protein or fragment thereof, an enzyme or a non-proteinaceous product of reactions of enzymes with a compound in the culture medium, or a low molecular weight product such as hormones or nucleic acids.

**35.** A method according to claim 34, in which the gene product is an enzyme.

**36.** A method according to claim 35, in which the enzyme is lipase, amyloglucosidase, $\alpha$-amylase, $\beta$-galactosidase, cellulase or proteases such as chymosin.

**37.** A method according to claim 32, in which the expression vector is a vector prepared according to any of claims 11-18.

**38.** A method according to claim 37, in which the fungus is a fungus of the genus *Mucor*.

EP 0 205 506 B1

**Revendications**
**Revendications pour les Etats contractants suivants : BE, CH, LI, DE, FR, GB, IT, LU, NL et SE**

**1.** Procédé de transformation d'un champignon de la classe des zygomycètes, dans lequel on transforme des sporangiospores ou jeunes germes d'un champignon de la classe des zygomycètes avec un vecteur d'expression recombinant qui est répliqué dans l'espèce fongique, la transformation étant effectuée en traitant des sporangiospores en germination avec une ou plusieurs enzymes lytiques appropriées, en transformant les protoplastes résultants avec un vecteur d'expression, et en régénérant la paroi cellulaire.

**2.** Procédé selon la revendication 1, dans lequel le champignon est un champignon du genre Mucor.

**3.** Procédé selon la revendication 2, dans lequel la souche de Mucor est sélectionnée parmi une espèce de Mucor mésophile telle que M. circinelloides, M. racemosus ou M. rouxii, ou une espèce de Mucor thermophile telle que M. miehei ou M. pusillus.

**4.** Procédé selon la revendication 1, dans lequel l'enzyme lytique est la chitosanase.

**5.** Procédé selon la revendication 1, dans lequel la souche fongique de la classe des Zygomycètes est une souche mutante sensible auxotrophe ou antibiotique qui, lorsqu'elle est transformée, est complémentée par un vecteur d'expression portant le gène de résistance prototrophique ou antibiotique contre-sélectible.

**6.** Procédé selon l'une quelconque des revendications précédentes, dans lequel le vecteur d'expression est auto-répliquant.

**7.** Procédé selon l'une quelconque des revendications 1-5, dans lequel le vecteur d'expression est intégré de façon stable dans un ou plusieurs des chromosomes hôtes.

**8.** Procédé selon l'une quelconque des revendications précédentes dans lequel le vecteur est un vecteur navette qui peut être répliqué dans une espèce fongique de la classe Zygomycetes, E. coli et/ou une levure.

**9.** Procédé selon la revendication 8, dans lequel le vecteur est pMCL 1302 porté dans Mucor circinelloides R7B, et déposé au Centraal Bureau voor Schimmelkulturen sous le N° CBS 754.84.

**10.** Procédé selon la revendication 8, dans lequel le vecteur est pMCL 1647, porté dans Mucor circinelloides R7B, et déposé au Centraal Bureau voor Schimmelkulturen sous le N° CBS 755.84.

**11.** Vecteur d'expression réplicable pour transformation d'un champignon de la classe des zygomycètes comprenant une séquence d'ADN provenant d'un champignon de la classe des zygomycètes qui comprend un marqueur sélectionnable, au moins un site de restriction pour l'insertion d'une séquence d'ADN codant pour un produit de gène désiré situé en une position dans laquelle l'insertion de la séquence d'ADN n'influera pas la réplication ou la sélection dudit vecteur,
et si on le désire une séquence codant pour la réplication autonome du vecteur dans un champignon de la classe des zygomycètes,
et que l'on peut obtenir par les étapes suivantes :
1) Préparation d'un plasmide recombinant, contenant un fragment d'ADN génomique d'un membre de la classe des zygomycètes et qui est réplicable dans une bactérie et/ou une levure, de façon connue ;
2) Transformation d'un mutant déficient d'un membre de la classe des zygomycètes avec ledit plasmide recombinant par traitement de sporangiospores germinant avec une ou plusieurs enzymes lytiques appropriées, transformation des protoplastes résultants avec ledit plasmide recombinant et régénération de la paroi cellulase, et
3) Sélection de mutants transformés et isolement à partir de ceux-ci desdits plasmides de façon connue.

**12.** Vecteur d'expression selon la revendication 11, dans lequel le marqueur est un gène qui joue un rôle

24

de médiation de la résistance antibiotique à une souche fongique sensible aux antibiotiques ou un gène jouant un rôle de médiateur de la prototrophie pour une souche fongique auxotrophe.

**13.** Vecteur d'expression selon la revendication 11 ou 12 qui est auto-répliquant.

**14.** Vecteur d'expression selon la revendication 11 ou 12 qui est intégré de façon stable dans un ou plusieurs des chromosomes hôtes.

**15.** Vecteur d'expression selon l'une quelconque des revendications 11-14 qui est un vecteur navette qui peut être répliqué dans une espèce de la classe Zygomycetes, E. coli et/ou une levure.

**16.** Vecteur d'expression selon la revendication 15, qui est pMCL 1302, porté dans Mucor circinelloides R7B, et déposé au Centraal Bureau voor Schimmelkulturen sous le N° CBS 754.84.

**17.** Vecteur d'expression selon la revendication 15, qui est pMCL 1647, porté dans Mucor circinelloides R7B, et déposé au Centraal Bureau voor Schimmelkulturen sous le N° CBS 755.84.

**18.** Champignon de la classe des zygomycètes qui porte un vecteur d'expression recombinant qui est répliqué dans l'organisme.

**19.** Champignon selon la revendication 18, dans lequel le vecteur d'expression est un vecteur selon l'une quelconque des revendications 31-38 ou 11-17.

**20.** Champignon selon la revendication 18 ou 19, qui est un champignon du genre Mucor.

**21.** Champignon selon la revendication 20, qui est une souche Mucor sélectionné parmi une espèce de Mucor mésophile telle que M. circinelloides, M. racemosus ou M. rouxii ou une espèce de Mucor thermophile telle que M. miehei ou M. pusillys.

**22.** Champignon selon l'une quelconque des revendications 18-21 qui est une souche mutante auxotrophe.

**23.** Procédé de préparation d'un vecteur d'expression selon l'une quelconque des revendications 31-38 dans lequel on insère, dans un plasmide qui est répliqué dans un champignon de la classe des zygomycètes, une séquence d'ADN pour un marqueur pour identification et/ou sélection de cellules transformées avec le vecteur, et l'on fournit un site de restriction pour l'insertion d'une séquence d'ADN codant pour un produit de gène désiré en une localisation où ladite séquence d'ADN ne gêne pas la réplication et/ou la sélection, et on insère ladite séquence d'ADN pour le produit de gène désiré en ce site.

**24.** Procédé de production d'un produit de gène provenant d'un champignon de la classe des zygomycètes, dans lequel on transforme des sporangiospores ou des jeunes germes d'un champignon de la classe des zygomycètes avec un vecteur d'expression recombinant qui est répliqué dans l'espèce fongique et qui porte une séquence d'ADN codant pour un produit de gène désiré selon le procédé de la revendication 1, les sporangiospores ou jeunes germes transformés sont cultivés dans un milieu de culture approprié pour exprimer ladite séquence d'ADN, et le produit résultant est recueilli.

**25.** Procédé selon la revendication 24, dans lequel le produit de l'ADN inséré est excrété dans le milieu et en est récupéré .

**26.** Procédé selon la revendication 24 ou 25 dans lequel le produit de gène produit à partir de l'espèce de zygomycète est un polypeptide, une protéine ou un de leurs fragments, une enzyme ou un produit non protéique de réactions d'enzymes avec un composé dans le milieu de culture ou un produit à faible poids moléculaire tel que des hormones ou des acides nucléiques.

**27.** Procédé selon la revendication 26 dans lequel le produit de gène est une enzyme.

**28.** Procédé selon la revendication 27 dans lequel l'enzyme est la lipase, l'amyloglucosidase, l' $\alpha$-amylase, la $\beta$-galactosidase, la cellulase ou des protéases telles que la chymosine.

**29.** Procédé selon la revendication 24 dans lequel le vecteur d'expression est un vecteur selon l'une quelconque des revendications 31-38.

**30.** Procédé selon la revendication 29 dans lequel le champignon est un champignon du genre Mucor.

**31.** Vecteur d'expression réplicable selon la revendication 11, qui contient en outre dans ledit site de restriction une séquence d'ADN qui peut être exprimée dans un champignon de la classe des zygomycètes et qui code pour un produit de gène désiré.

**32.** Vecteur d'expression selon la revendication 31, dans lequel le marqueur est un gène jouant un rôle de médiation de la résistance antibiotique à une souche fongique sensible aux antibiotiques ou un gène jouant un rôle de médiation de la prototrophie vis-à-vis d'une souche fongique auxotrophe.

**33.** Vecteur d'expression selon la revendication 31 ou 32 qui est auto-répliquant.

**34.** Vecteur d'expression selon la revendication 31 ou 32 qui est intégré de façon stable dans un ou plusieurs des chromosomes hôtes.

**35.** Vecteur d'expression selon l'une quelconque des revendications 31-34 qui est un vecteur navette qui peut être répliqué dans une espèce fongique de la classe des zygomycètes, E. coli et/ou une levure.

**36.** Vecteur d'expression selon l'une quelconque des revendications 31-35, dans lequel le produit de gène encodé par la séquence d'ADN est un polypeptide ou protéine ou un de leurs fragments, une enzyme ou un produit non protéique de réactions d'enzymes avec un composé dans le milieu de culture, ou un produit à faible poids moléculaire tel que des hormones ou des acides nucléiques.

**37.** Vecteur d'expression selon la revendication 36 dans lequel le produit de gène est une enzyme.

**38.** Vecteur d'expression selon la revendication 37 dans lequel l'enzyme est la lipase, l'amyloglucosidase, l'$\alpha$-amylase, la $\beta$-galactosidase, la cellulase ou une protéase telle que la chymosine.

**Revendications pour l'Etat contractant suivant : AT**

**1.** Procédé de transformation d'un champignon de la classe des zygomycètes, dans lequel on transforme des sporangiospores ou des jeunes germes d'un champignon de la classe des zygomycètes avec un vecteur d'expression recombinant qui est répliqué dans l'espèce fongique, la transformation s'effectuant en traitant des sporangiospores en germination avec une ou plusieurs enzymes lytiques appropriées, en transformant les protoplastes résultants avec un vecteur d'expression, et en régénérant la paroi cellulaire.

**2.** Procédé selon la revendication 1, dans lequel le champignon est un champignon du genre Mucor.

**3.** Procédé selon la revendication 2, dans lequel la souche de Mucor est sélectionnée dans une espèce de Mucor mésophile telle que M. circinelloides, M. racemosus ou M. rouxii, ou une espèce de Mucor thermophile telle que M. miehei ou M. pusillus.

**4.** Procédé selon la revendication 1, dans lequel l'enzyme lytique est la chitosanase.

**5.** Procédé selon la revendication 1, dans lequel la souche fongique de la classe des zygomycètes est une souche mutante auxotrophe ou sensible aux antibiotiques qui, lorsqu'elle est transformée, est complémentée par un vecteur d'expression portant le gène prototrophe ou de résistance aux antibiotiques contre-sélectible.

**6.** Procédé selon l'une quelconque des revendications précédentes, dans lequel le vecteur d'expression est auto-répliquant.

**7.** Procédé selon l'une quelconque des revendications 1-5, dans lequel le vecteur d'expression est intégré de façon stable dans un ou plusieurs des chromosomeshôtes.

**8.** Procédé selon l'une quelconque des revendications précédentes dans lequel le vecteur est un vecteur navette qui peut être répliqué dans une espèce fongique de la classe des zygomycètes, E. coli et/ou une levure.

**9.** Procédé selon la revendication 8, dans lequel le vecteur est pMCL 1302, porté dans Mucor circinelloides R7B, et déposé au Centraal Bureau voor Schimmelkulturen sous le N° CBS 754.84.

**10.** Procédé selon la revendication 8, dans lequel le vecteur est pMCL 1647, porté dans Mucor circinelloides R7B, et déposé au Centraal Bureau voor Schimmelkulturen sous le N° CBS 755.84.

**11.** Procédé de préparation d'un vecteur d'expression pour la transformation d'un champignon de la classe des zygomycètes par combinaison d'une séquence d'ADN codant pour un produit de gène désiré, et d'un marqueur pour l'identification et/ou la sélection de cellules transformées avec le vecteur, le vecteur étant répliqué dans des champignons de la classe des zygomycètes.

**12.** Procédé selon la revendication 11, dans lequel le marqueur est un gène jouant un rôle de médiation de la résistance aux antibiotiques pour une souche fongique sensible aux antibiotiques ou un gène jouant un rôle de médiateur de la prototrophie pour une souche fongique auxotrophe.

**13.** Procédé selon la revendication 11 ou 12 qui est auto-répliquant.

**14.** Procédé selon la revendication 11 ou 12 qui est intégré de façon stable dans un ou plusieurs des chromosomes hôtes.

**15.** Procédé selon l'une quelconque des revendications 11-14 comportant un vecteur navette qui peut être répliqué dans une espèce fongique de la classe des zygomycètes, E. coli et/ou une levure.

**16.** Procédé selon l'une des revendications 11-15, dans lequel le produit de gène encodé par la séquence d'ADN est un polypeptide ou une protéine ou un de leurs fragments, une enzyme ou un produit non-protéique de réactions d'enzymes avec un composé dans le milieu de culture, ou un produit à faible poids moléculaire comme des hormones ou des acides nucléiques.

**17.** Procédé selon la revendication 16 , dans lequel le produit de gène est une enzyme.

**18.** Procédé selon la revendication 17, dans lequel l'enzyme est la lipase, l'amyloglucosidase, l' $\alpha$-amylase, la $\beta$-galactosidase, la cellulase ou une protéase comme la chymosine.

**19.** Procédé de préparation d'un vecteur d'expression pour la transformation d'un champignon de la classe des zygomycètes par combinaison d'un marqueur pour l'identification et/ou la sélection de cellules transformées avec le vecteur, et au moins un site de restriction pour l'insertion d'une séquence d'ADN codant pour un produit de gène désiré, le vecteur étant répliqué dans des champignons de la classe des zygomycètes.

**20.** Procédé selon la revendication 19, dans lequel le marqueur est un gène jouant un rôle de médiateur de la résistance aux antibiotiques pour une souche fongique sensible aux antibiotiques ou un gène jouant un rôle de médiateur de la prototrophie pour une souche fongique auxotrophe.

**21.** Procédé selon la revendication 19 ou 20 qui est auto-répliquant.

**22.** Procédé selon la revendication 19 ou 20 qui est intégré de façon stable dans un ou plusieurs des chromosomes hôtes.

**23.** Procédé selon l'une quelconque des revendications 19-22 qui comprend un vecteur navette pouvant être répliqué dans une espèce de la classe des zygomycètes, E; coli et/ou une levure.

**24.** Procédé selon la revendication 23, incluant pMCL 1302, porté dans Mucor circinelloides R7B, et déposé au Centraal Bureau voor Schimmelkulturen sous le N° CBS 754.84.

**25.** Procédé selon la revendication 23, incluant pMCL 1647, porté dans Mucor circinelloides R7B, et déposé au Centraal Bureau voor Schimmelkulturen sous le N° CBS 755.84.

**26.** Procédé de préparation d'un champignon de la classe des zygomycètes par introduction d'un vecteur d'expression recombinant qui est répliqué dans l'organisme.

**27.** Procédé selon la revendication 26, dans lequel le vecteur d'expression est un vecteur selon l'une quelconque des revendications 11-18 ou 19-25.

**28.** Procédé selon la revendication 26 ou 27, incluant un champignon du genre Mucor.

**29.** Procédé selon la revendication 28, qui est une souche de Mucor sélectionnée dans une espèce de Mucor mésophile telle que M. circinelloides, M. racemosus ou M. rouxii ou une espèce de Mucor thermophile telle que M. miehei ou M. pusillys.

**30.** Procédé selon l'une quelconque des revendications 26-29 incluant une souche mutante auxotrophe.

**31.** Procédé de préparation d'un vecteur d'expression selon l'une quelconque des revendications 11-18, dans lequel on insère, dans un plasmide qui est répliqué dans un champignon de la classe des zygomycètes, une séquence d'ADN pour un marqueur pour l'identification et/ou la sélection de cellules transformées avec le vecteur, et l'on fournit un site de restriction pour l'insertion d'une séquence d'ADN codant pour un produit de gène désiré en une localisation dans laquelle ladite séquence d'ADN ne gène pas la réplication et/ou la sélection, et on insère ladite séquence d'ADN pour le produit de gène désiré en ce site.

**32.** Procédé de préparation d'un produit de gène à partir d'un champignon de la classe des zygomycètes, dans lequel on transforme des sporangiospores ou des jeunes germes d'un champignon de la classe des zygomycètes avec un vecteur d'expression recombinant qui est répliqué dans l'espèce fongique et qui porte une séquence d'ADN codant pour un produit de gène désiré selon la procédé de la revendication 1, on cultive les sporangiospores ou jeunes germes transformés dans un milieu de culture approprié pour exprimer ladite séquence d'ADN, et on recueille le produit résultant.

**33.** Procédé selon la revendication 32, dans lequel le produit de l'ADN inséré est excrété dans le milieu et recueilli à partir de ce milieu.

**34.** Procédé selon la revendication 32 ou 33, dans lequel le produit de gène préparé à partir de l'espèce de zygomycète est un polypeptide ou une protéine ou un de leurs fragments, une enzyme ou un produit non-protéique de réactions d'enzymes avec un composé dans le milieu de culture, ou un produit de faible poids moléculaire corne des hormones ou des acides nucléiques.

**35.** Procédé selon la revendication 34, dans lequel le produit de gène est une enzyme.

**36.** Procédé selon la revendication 35, dans lequel l'enzyme est la lipase, l'amyloglucosidase, l' $\alpha$-amylase, la $\beta$-galactosidase, la cellulase ou une protéase telle que la chymosine.

**37.** Procédé selon la revendication 32 dans lequel le vecteur d'expression des un vecteur préparé selon l'une quelconque des revendications 11-18.

**38.** Procédé selon la revendication 37, dans lequel le champignon est un champignon du genre Mucor.

**Patentansprüche**
**Patentansprüche für folgende Vertragsstaaten : BE, CH, LI, DE, FR, GB, IT, LU, NL und SE**

**1.** Ein Verfahren zum Transformieren eines Pilzes der Klasse Zygomycetes, bei dem Sporangiosporen oder Keimlinge eines Pilzes der Klasse Zygomycetes mit einem rekombinanten Expressionsvektor transformiert werden, der in der Pilzart repliziert wird, wobei die Transformation erfolgt durch Behandeln von keimenden Sporangiosporen mit einem oder mehreren geeigneten lytischen Enzymen, Transformiern der erhaltenen Protoplasten mit einem Expressionsvektor und Regenerieren der Zellwand.

28

**2.** Verfahren gemäß Anspruch 1, bei dem der Pilz ein Pilz der Gattung Mucor ist.

**3.** Ein Verfahren gemäß Anspruch 2, in dem der Mucor-Stamm ausgewählt wird aus einer mesophilen Mucor-Art, wie M. circinelloides, M. racemosus oder M. rouxii, oder einer thermophilen Mucor-Art, wie M. miehei oder M. pusillus.

**4.** Ein Verfahren gemäß Anspruch 1, in dem das lytische Enzym Chitosanase ist.

**5.** Ein Verfahren gemäß Anspruch 1, in dem der Pilzstamm der Klasse Zygomycetes ein geeigneter auxotropher oder antibiotikumsensitiver Mutantenstamm ist, der, wenn transformiert, komplementiert wird durch einen Expressionsvektor, der das gegenselektierbare, prototrophe oder Antibiotikumresistenzgen trägt.

**6.** Ein Verfahren gemäß einem der vorhergehenden Ansprüche, in dem der Expressionsvektor selbstreplizierend ist.

**7.** Ein Verfahren gemäß einem der Ansprüche 1 bis 5, in dem der Expressionsvektor stabil in eines oder mehrere der Wirtschromosomen integriert wird.

**8.** Ein Verfahren gemäß einem der vorhergehenden Ansprüche, in dem der Vektor ein Shuttle-Vektor ist, der in einer Pilzart der Klasse Zygomycetes, E. coli und/oder einer Hefe repliziert werden kann.

**9.** Ein Verfahren gemäß Anspruch 8, in dem der Vektor pMCL 1302, enthaltend in Mucor circinelloides R7B, und hinterlegt bei dem Centraal Bureau voor Schimmelkulturen unter der Hinterlegungsnummer CBS 754.84, ist.

**10.** Ein Verfahren gemäß Anspruch 8, in dem der Vektor pMCL 1647, enthaltend in Mucor circinelloides R7B, hinterlegt bei dem Centraal Bureau voor Schimmelkulturen unter der Hinterlegungsnummer CBS 755.84, ist.

**11.** Ein replizierbarer Expressionsvektor zur Transformation eines Pilzes der Klasse Zygomycetes, umfassend

eine DNA-Sequenz von einem Pilz der Klasse Zygomycetes, die umfaßt einen selektierbaren Marker,

mindestens eine Restriktionsspaltstelle für den Einbau einer DNA-Sequenz, die für ein gewünschtes Genprodukt codiert, die an einer Position gelegen ist, wo der Einbau der DNA-Sequenz nicht die Replikation oder Selektion des Vektors beinflußt,

und gegebenenfalls eine Sequenz, die für die autonome Replikation des Vektors in einem Pilz der Klasse Zygomycetes codiert,

und der erhältlich ist durch die folgenden Schritte:
1) Herstellen eines rekombinanten Plasmids, enthaltend ein genomisches DNA-Fragment eines Mitglieds der Klasse Zygomycetes, und das in einem Bakterium und/oder einer Hefe replizierbar ist, in an sich bekannter Art und Weise,
2) Transformieren einer Mangelmutante eines Mitglieds der Klasse Zygomycetes mit dem rekombinanten Plasmid durch Behandeln von keimenden Sporangiosporen mit einem oder mehreren geeigneten lytischen Enzymen, Transformieren der erhaltenen Protoplasten mit dem rekombinanten Plasmid und Regenerieren der Zellwand, und
3) Selektieren von transformierten Mutanten und Isolieren der Plasmide aus diesen in an sich bekannter Art und Weise.

**12.** Ein Expressionsvektor gemäß Anspruch 11, in dem der Marker ein Gen ist, das einem antibiotikasensitiven Pilzstamm Antibiotikaresistenz vermittelt, oder ein Gen ist, das einem auxotrophen Pilzstamm Prototrophie vermittelt.

**13.** Ein Expressionsvektor gemäß Anspruch 11 oder 12, der selbstreplizierend ist.

**14.** Ein Expressionsvektor gemäß Anspruch 11 oder 12, der stabil in eines oder mehrere der Wirtschromosomen integriert ist.

**15.** Ein Expressionsvektor gemäß einem der Ansprüche 11 bis 14, der ein Shuttle-Vektor ist, der in einer Art der Klasse Zygomycetes, E. coli und/oder einer Hefe repliziert werden kann.

**16.** Ein Expressionsvektor gemäß Anspruch 15, der pMCL 1302, enthaltend in Mucor circinelloides R7B, und hinterlegt bei dem Centraal Bureau voor Schimmelkulturen unter der Hinterlegungsnummer CBS 754.84, ist.

**17.** Ein Expressionsvektor gemäß Anspruch 15, der pMCL 1647, enthaltend in Mucor circinelloides R7B, und hinterlegt bei dem Centraal Bureau voor Schimmelkulturen unter der Hinterlegungsnummer CBS 755.84, ist.

**18.** Ein Pilz der Klasse Zygomycetes, der einen rekombinanten Expressionsvektor trägt, der in dem Organismus repliziert wird.

**19.** Ein Pilz gemäß Anspruch 18, in dem der Expressionsvektor ein Vektor gemäß einem der Ansprüche 31 bis 38 oder 11 bis 17 ist.

**20.** Ein Pilz gemäß Anspruch 18 oder 19, der ein Pilz der Gattung Mucor ist.

**21.** Ein Pilz gemäß Anspruch 20, der ein Mucor-Stamm ist ausgewählt aus einer mesophilen Mucor-Art, wie M. circinelloides, M. racemosus oder M. rouxii, oder einer thermophilen Mucor-Art, wie M. miehei oder M. pusillys.

**22.** Ein Pilz gemäß einem der Ansprüche 18 bis 21, der ein auxotropher Mutantenstamm ist.

**23.** Ein Verfahren zum Herstellen eines Expressionsvektors gemäß einem der Ansprüche 31 bis 38, umfassend Einfügen in ein Plasmid, das in einem Pilz der Klasse Zygomycetes repliziert wird, einer DNA-Sequenz für einen Marker für die Identifizierung und/oder Selektion von Zellen, die mit dem Vektor transformiert sind, und Bereitstellen einer Restriktionsspaltstelle für den Einbau einer DNA-Sequenz, die für ein gewünschtes Produkt codiert, in einer Position, wo die DNA-Sequenz nicht die Replikation und/oder Selektion beeinflußt, und Einbauen der DNA-Sequenz für das gewünschte Produkt an dieser Stelle.

**24.** Ein Verfahren zum Herstellen eines Genprodukts aus einem Pilz der Klasse Zygomycetes, in dem Sporangiosporen oder Keimlinge eines Pilzes der Klasse Zygomycetes gemäß dem Verfahren von Anspruch 1 mit einem rekombinanten Expressionsvektor, der in der Pilzart repliziert wird, und der eine DNA-Sequenz trägt, die für ein gewünschtes Genprodukt codiert, transformiert werden, die transformierten Sporangiosporen oder Keimlinge werden in einem geeigneten Kulturmedium kultiviert, um die DNA-Sequenz zu exprimieren, und das erhaltene Produkt wird geerntet.

**25.** Ein Verfahren nach Anspruch 24, in dem das Produkt der eingebauten DNA in das Medium ausgeschieden und von diesem wiedergewonnen wird.

**26.** Ein Verfahren gemäß Anspruch 24 oder 25, in dem das von der Zygomycetes-Art erzeugte Produkt ein Polypeptid oder Protein oder Fragment davon, ein Enzym oder ein nichtproteinartiges Produkt von Reaktionen von Enzymen mit einem Bestandteil in dem Kulturmedium oder ein Produkt mit niedrigem Molekulargewicht, wie Hormone oder Nukleinsäuren, ist.

**27.** Ein Verfahren gemäß Anspruch 26, in dem das Genprodukt ein Enzym ist.

**28.** Ein Verfahren gemäß Anspruch 27, in dem das Enzym Lipase, Amyloglucosidase, $\alpha$-Amylase, $\beta$-Galactosidase, Zellulase oder Proteasen, wie Chymosin, ist.

**29.** Ein Verfahren gemäß Anspruch 24, in dem der Expressionsvektor ein Vektor gemäß einem der Ansprüche 31 bis 38 ist.

30

**30.** Ein Verfahren gemäß Anspruch 29, in dem der Pilz ein Pilz der Gattung Mucor ist.

**31.** Ein replizierbarer Expressionsvektor gemäß Anspruch 11, der weiterhin in der Restriktionsspaltstelle eine DNA-Sequenz enthält, die in einem Pilz der Klasse Zygomycetes exprimiert werden kann und die für ein gewünschtes Genprodukt codiert.

**32.** Ein Expressionsvektor gemäß Anspruch 31, in dem der Marker ein Gen ist, das einem antibiotikasensitiven Pilzstamm Antibiotikaresistenz verleiht, oder ein Gen ist, das einem auxotrophen Pilzstamm Prototrophie verleiht.

**33.** Ein Expressionsvektor gemäß Anspruch 31 oder 32, der selbstreplizierend ist.

**34.** Ein Expressionsvektor gemäß Anspruch 31 oder 32, der stabil in eins oder mehrere der Wirtschromosomen integriert ist.

**35.** Ein Expressionsvektor gemäß einem der Ansprüche 31 bis 34, der ein Shuttle-Vektor ist, der in einer Pilzart der Klasse Zygomycetes, E. coli und/oder einer Hefe repliziert werden kann.

**36.** Ein Expressionsvektor gemäß einem der Ansprüche 31 bis 35, in dem das durch die DNA-Sequenz codierte Genprodukt ein Polypeptid oder Protein oder Fragment davon, ein Enzym oder ein nichtproteinartiges Produkt von Reaktionen von Enzymen mit einem Bestandteil in dem Kulturmedium oder ein Produkt mit niedrigem Molekulargewicht, wie Hormone oder Nukleinsäuren, ist.

**37.** Ein Expressionsvektor gemäß Anspruch 36, in dem das Genprodukt ein Enzym ist.

**38.** Ein Expressionsvektor gemäß Anspruch 37, in dem das Enzym Lipase, Amyloglucosidase, $\alpha$-Amylase, $\beta$-Galactosidase, Zellulase oder Protease, wie Chymosin, ist.

**Patentansprüche für folgenden Vertragsstaat : AT**

**1.** Ein Verfahren zum Transformieren eines Pilzes der Klasse Zygomycetes, bei dem Sporangiosporen oder Keimlinge eines Pilzes der Klasse Zygomycetes mit einem rekombinanten Expressionsvektor transformiert werden, der in der Pilzart repliziert wird, wobei die Transformation erfolgt durch Behandeln von keimenden Sporangiosporen mit einem oder mehreren geeigneten lytischen Enzymen, Transformiern der erhaltenen Protoplasten mit einem Expressionsvektor und Regenerieren der Zellwand.

**2.** Verfahren gemäß Anspruch 1, bei dem der Pilz ein Pilz der Gattung Mucor ist.

**3.** Ein Verfahren gemäß Anspruch 2, in dem der Mucor-Stamm ausgewählt wird aus einer mesophilen Mucor-Art, wie M. circinelloides, M. racemosus oder M. rouxii, oder einer thermophilen Mucor-Art, wie M. miehei oder M. pusillus.

**4.** Ein Verfahren gemäß Anspruch 1, in dem das lytische Enzym Chitosanase ist.

**5.** Ein Verfahren gemäß Anspruch 1, in dem der Pilzstamm der Klasse Zygomycetes ein geeigneter auxotropher oder antibiotikumsensitiver Mutantenstamm ist, der, wenn transformiert, komplementiert wird durch einen Expressionsvektor, der das gegenselektierbare, prototrophe oder Antibiotikumresistenzgen trägt.

**6.** Ein Verfahren gemäß einem der vorhergehenden Ansprüche, in dem der Expressionsvektor selbstreplizierend ist.

**7.** Ein Verfahren gemäß einem der Ansprüche 1 bis 5, in dem der Expressionsvektor stabil in eines oder mehrere der Wirtschromosomen integriert wird.

**8.** Ein Verfahren gemäß einem der vorhergehenden Ansprüche, in dem der Vektor ein Shuttle-Vektor ist, der in einer Pilzart der Klasse Zygomycetes, E. coli und/oder einer Hefe repliziert werden kann.

**9.** Ein Verfahren gemäß Anspruch 8, in dem der Vektor pMCL 1302, enthaltend in Mucor circinelloides R7B, und hinterlegt bei dem Centraal Bureau voor Schimmelkulturen unter der Hinterlegungsnummer CBS 754.84, ist.

**10.** Ein Verfahren gemäß Anspruch 8, in dem der Vektor pMCL 1647, enthaltend in Mucor circinelloides R7B, hinterlegt bei dem Centraal Bureau voor Schimmelkulturen unter der Hinterlegungsnummer CBS 755.84, ist.

**11.** Ein Verfahren zum Herstellen eines Expressionsvektors zum Transformieren eines Pilzes der Klasse Zygomycetes durch Kombinieren einer DNA-Sequenz, die für ein gewünschtes Genprodukt codiert, und eines Markers für die Identifizierung und/oder Selektion von mit dem Vektor transformierten Zellen, wobei der Vektor in Pilzen der Klasse Zygomycetes repliziert wird.

**12.** Ein Verfahren gemäß Anspruch 11, in dem der Marker ein Gen ist, das einem antibiotikasensitiven Pilzstamm Antibiotikaresistenz verleiht, oder ein Gen ist, das einem auxotrophen Pilzstamm Prototrophie verleiht.

**13.** Ein Verfahren gemäß Anspruch 11 oder 12, in dem der Expressionsvektor selbstreplizierend ist.

**14.** Ein Verfahren gemäß Anspruch 11 oder 12, in dem der Expressionsvektor stabil in eines oder mehrere der Wirtschromosomen integriert wird.

**15.** Ein Verfahren gemäß einem der Ansprüche 11 bis 14, in dem der Expressionsvektor ein Shuttle-Vektor ist, der in einer Pilzart der Klasse Zygomycetes, E. coli und/oder einer Hefe repliziert werden kann.

**16.** Ein Verfahren gemäß einem der Ansprüche 11 bis 15, in dem das durch die DNA-Sequenz codierte Genprodukt ein Polypeptid oder ein Protein oder Fragment davon, ein Enzym oder ein nichtproteinartiges Produkt von Reaktionen von Enzymen mit einem Bestandteil in dem Kulturmedium oder ein Produkt mit niedrigem Molekulargewicht, wie Hormone oder Nukleinsäuren, ist.

**17.** Ein Verfahren gemäß Anspruch 16, in dem das Genprodukt ein Enzym ist.

**18.** Ein Verfahren gemäß Anspruch 17, in dem das Enzym Lipase, Amyloglucidase, $\alpha$-Amylase, $\beta$-Galactosidase, Zellulase oder Protease, wie Chymosin, ist.

**19.** Ein Verfahren zum Herstellen eines Expressionsvektors für die Transformation eines Pilzes der Klasse Zygomycetes durch Kombinieren eines Markers für die Identifizierung und/oder Selektion von Zellen, die mit dem Vektor transformiert sind, und mindestens einer Restriktionsspaltstelle für den Einbau einer DNA-Sequenz, die für ein gewünschtes Genprodukt codiert, wobei der Vektor in Pilzen der Klasse Zygomycetes repliziert wird.

**20.** Ein Verfahren gemäß Anspruch 19, in dem der Marker ein Gen ist, das einem antibiotikasensitiven Pilzstamm Antibiotikaresistenz vermittelt, oder ein Gen ist, das einem auxotrophen Pilzstamm Prototrophie vermittelt.

**21.** Ein Verfahren gemäß Anspruch 19 oder 20, in dem der Expressionsvektor selbstreplizierend ist.

**22.** Ein Verfahren gemäß Anspruch 19 oder 20, in dem der Expressionsvektor stabil in eines oder mehrere der Wirtschromosomen integriert wird.

**23.** Ein Verfahren gemäß einem der Ansprüche 19 bis 22, in dem der Expressionsvektor ein Shuttle-Vektor ist, der in einer Art der Klasse Zygomycetes, E. coli und /oder einer Hefe repliziert werden kann.

**24.** Ein Verfahren gemäß Anspruch 23, in dem der Expressionsvektor pMCL 1302, enthaltend in Mucor circinelloides R7B, und hinterlegt bei dem Centraal Bureau voor Schimmelkulturen unter der Hinterlegungsnummer CBS 754.84, ist.

**25.** Ein Verfahren gemäß Anspruch 23, in dem der Expressionsvektor pMCL 1647, enthaltend in Mucor circinelloides R7B, und hinterlegt bei dem Centraal Bureau voor Schimmelkulturen unter der Hinterlegungsnummer CBS 755.84, ist.

**26.** Ein Verfahren zum Herstellen eines Pilzes der Klasse Zygomycetes durch Einführen eines rekombinanten Expressionsvektors, der in dem Organismus repliziert wird.

**27.** Ein Verfahren gemäß Anspruch 26, in dem der Expressionsvektor ein Vektor gemäß einem der Ansprüche 11 bis 18 oder 19 bis 25 ist.

**28.** Ein Verfahren gemäß Anspruch 26 oder 27, in dem der Pilz ein Pilz der Gattung Mucor ist.

**29.** Ein Verfahren gemäß Anspruch 28, in dem der Pilz ein Mucor-Stamm ist, ausgewählt aus einer mesophilen Mucor-Art, wie M. circinelloides, M. racemosus oder M. rouxii, oder einer thermophilen Mucor-Art, wie M. miehei oder M. pusillys.

**30.** Ein Verfahren gemäß einem der Ansprüche 26 bis 29, in dem der Pilz ein auxotropher Mutantenstamm ist.

**31.** Ein Verfahren zum Herstellen eines Expressionsvektors gemäß einem der Ansprüche 11 bis 18, umfassend Einfügen in ein Plasmid, das in einem Pilz der Klasse Zygomycetes repliziert wird, einer DNA-Sequenz für einen Marker für die Identifizierung und/oder Selektion von Zellen, die mit dem Vektor transformiert sind, und Bereitstellen einer Restriktionsspaltstelle für den Einbau einer DNA-Sequenz, die für ein gewünschtes Genprodukt codiert, in einer Position, wo die DNA-Sequenz nicht die Replikation und/oder Selektion beeinflußt, und Einbauen der DNA-Sequenz für das gewünschte Genprodukt an dieser Stelle.

**32.** Ein Verfahren zum Herstellen eines Genprodukts aus einem Pilz der Klasse Zygomycetes, in dem Sporangiosporen oder Keimlinge eines Pilzes der Klasse Zygomycetes gemäß dem Verfahren von Anspruch 1 mit einem rekombinanten Expressionsvektor, der in der Pilzart repliziert wird, und der eine DNA-Sequenz trägt, die für ein gewünschtes Genprodukt codiert, transformiert werden, die transformierten Sporangiosporen oder Keimlinge werden in einem geeigneten Kulturmedium kultiviert, um die DNA-Sequenz zu exprimieren und das erhaltene Produkt wird geernted.

**33.** Ein Verfahren gemäß Anspruch 32, in dem das Produkt der eingebauten DNA in das Medium ausgeschieden und von diesem wiedergewonnen wird.

**34.** Ein Verfahren gemäß Anspruch 32 oder 33, in dem das von der Zygomycetes-Art erzeugte Produkt ein Polypeptid oder Protein oder Fragment davon, ein Enzym oder ein nichtproteinartiges Produkt von Reaktionen von Enzymen mit einem Bestandteil in dem Kulturmedium oder ein Produkt mit niedrigem Molekulargewicht, wie Hormone oder Nukleinsäuren, ist.

**35.** Ein Verfahren gemäß Anspruch 34, in dem das Genprodukt ein Enzym ist.

**36.** Ein Verfahren gemäß Anspruch 35, in dem das Enzym Lipase, Amyloglucosidase, $\alpha$-Amylase, $\beta$-Galactosidase, Zellulase oder Proteasen, wie Chymosin, ist.

**37.** Ein Verfahren gemäß Anspruch 32, in dem der Expressionsvektor ein Vektor ist, der gemäß einem der Ansprüche 11 bis 18 hergestellt worden ist.

**38.** Ein Verfahren gemäß Anspruch 37, in dem der Pilz ein Pilz der Gattung Mucor ist.

Fig. 1.

Fig. 2.

EP 0 205 506 B1

Fig. 3